# EUROPÄISCHE PATENTANMELDUNG

(11) **EP 4 552 601 A1**
(43) Veröffentlichungstag der Anmeldung: **14.05.2025**
(21) Anmeldenummer: 24211826.3
(22) Anmeldetag: 08.11.2024
(51) Int. Cl.: A61B 50/33, A61B 90/90, A61B 90/40, A61B 50/00, A61B 50/15, A61B 90/96

(54) **UNTERSTÜTZUNG DER HANDHABUNG MEDIZINISCHER INSTRUMENTE**

(30) Priorität: 13.11.2023 DE 102023131407
(71) Anmelder: Karl Storz SE & Co. KG, 78532 Tuttlingen (DE)
(72) Erfinder: Irion, Klaus M., 78532 Tuttlingen (DE)

(57) **Zusammenfassung**

Ein System zur Unterstützung der Handhabung medizinischer Instrumente (10) umfasst eine Kamera (42, 52, 62) zum optischen Erfassen eines Bilds eines oder mehrerer medizinischer Instrumente (10) und zum Bereitstellen eines Bildsignals, das das erfasste Bild repräsentiert, eine Bildauswertungseinrichtung (70) zum Empfangen des Bildsignals, zum Identifizieren von einem oder mehreren in dem durch das Bildsignal repräsentierten Bild abgebildeten medizinischen Instrumenten (10) und zum Bereitstellen von Identifikationsdaten, die die abgebildeten Instrumente (10) identifizieren, und eine Datenübertragungseinrichtung (56, 66) zum Senden von Identifikationsdaten an eine Datenempfangseinrichtung (88) einer mit dem autoklavierbaren Instrumententräger (20) dauerhaft verbundenen Dateneinrichtung (22).

## Beschreibung

Die vorliegende Erfindung ist auf ein System, einen autoklavierbaren Instrumententräger, einen autoklavierbaren Sterilbehälter und ein Verfahren zur Unterstützung der Handhabung medizinischer Instrumente bezogen.

In WO 2009/003231 A1 (Mems-ID Pty Ltd.) ist ein System zum Identifizieren chirurgischer Instrumente beschrieben. Die chirurgischen Instrumente sind mit RFID-Etiketten, die eine Sterilisation überstehen, versehen. An einem Behälter, der ein Tablett sein kann, zur Aufnahme der chirurgischen Instrumente ist eine abnehmbare Abfragevorrichtung vorgesehen, mittels derer die RFID-Etiketten an chirurgischen Instrumenten in dem Behälter abgefragt werden. Die Abfragevorrichtung wird vor der Sterilisation von dem Behälter abgenommen und danach wieder an dem Behälter angebracht.

In WO 2009/076452 A2 ist (Robotic Systems & Tevchnologies, Inc.; auch veröffentlicht als US 2011/0005342 A1, US 2011/0262250 A1, US 7,997,847 B2,

US 8,567,880 B2) ist ein Verfahren zum Handhaben einer Mehrzahl von chirurgischen Instrumenten zur Reinigung beschrieben. Ein Einsatz mit einer vorbestimmten Konfiguration zur Aufnahme mindestens eines Typs chirurgischer Instrumente wird identifiziert. Mittels einer optischen Vorrichtung oder eines RFID-Lesers wird jeder Typ der Mehrzahl chirurgischer Instrumente identifiziert. Mittels einer automatisierten Vorrichtung wird jedes der identifizierten chirurgischen Instrumente orientiert. Mittels der automatisierten Vorrichtung wird jeder Typ chirurgisches Instrument in einem oder mehreren Bereichen des Einsatzes angeordnet. In US 2016/0085922 A1 (Spinal Generations, LLC; auch veröffentlicht als

US 10,552,574 B2) ist das Identifizieren einer medizinischen Vorrichtung beschrieben. Die medizinische Vorrichtung wird optisch mittels einer Kamera und Bilderkennung und/oder mittels eines RFID-Lesers, der ein RFID-Etikett an der medizinischen Vorrichtung ausliest, identifiziert. Der RFID-Leser ist insbesondere in einem Operationssaal angeordnet. Ein Tablett weist ein RFID-Etikett auf, das Information bezüglich des Inhalts des Tabletts enthält.

In DE 10 2015 108 264 A1 (Aesculap AG; auch veröffentlicht als WO 2016/188959 A1, EP 3 302 340 B1, US 2018/0153639 A1, US 10,368,958 B2, CN 107708599 A) ist ein chirurgisches Behälterinhalt-Erfassungssystem beschrieben. In einem Sterilisationsbehälter ist ein Träger in Form einer Matte angeordnet. Der Träger umfasst ein Trägermodul mit einer Transponder-Leseeinrichtung, insbesondere einem RFID-Lesegerät, einer Speichereinrichtung zur Zwischenspeicherung erfasster Daten, einem Energiespeicher zur Eigenversorgung des Trägermoduls und einer drahtlos arbeitenden Datenübermittlungseinrichtung. Das RFID-Lesegerät erfasst Identifizierungselemente an Gegenständen in dem Sterilisationsbehälter. Information über den durch das erfasste Indentifizierungselement identifizierten Gegenstand wird von der drahtlos arbeitenden Datenübermittlungseinrichtung drahtlos nach außerhalb des Sterilisationsbehälter übertragen.

In WO 2016/023097 A1 (Synaptive Medical, Inc.; auch veröffentlicht als CA 2 957 794 A1, CA 2 957 794 C, US 2017/0243157 A1, US 10,592,857 B2) sind ein System und ein Verfahren zum Verwalten von Ausrüstungsgegenständen für eine medizinische Anwendung beschrieben. Eine Eingangsvorrichtung zum Identifizieren von Ausrüstungsgegenständen umfasst eine Verfolgungskamera, eine optische Kamera, einen RFID-Empfänger, eine Strukturlichtkamera oder ein Stereokamerapaar.

Eine Aufgabe der vorliegenden Erfindung besteht darin, ein verbessertes System, einen verbesserten autoklavierbaren Instrumententräger, einen verbesserten autoklavierbaren Sterilbehälter und ein verbessertes Verfahren zur Unterstützung der Handhabung medizinischer Instrumente zu schaffen.

Diese Aufgabe wird gelöst durch die Gegenstände der unabhängigen Ansprüche.

Weitere Ausführungsformen sind in den abhängigen Ansprüchen definiert.

Ein System zur Unterstützung der Handhabung medizinischer Instrumente umfasst eine Kamera zum optischen Erfassen eines Bilds eines oder mehrerer medizinischer Instrumente und zum Bereitstellen eines Bildsignals, das das erfasste Bild repräsentiert, eine Bildauswertungseinrichtung zum Empfangen des Bildsignals, zum Identifizieren von einem oder mehreren in dem durch das Bildsignal repräsentierten Bild abgebildeten medizinischen Instrumenten und zum Bereitstellen von Identifikationsdaten, die die abgebildeten Instrumente identifizieren, und eine Datenübertragungseinrichtung zum Senden von Identifikationsdaten an eine Datenempfangseinrichtung einer mit einem autoklavierbaren Instrumententräger dauerhaft verbundenen Dateneinrichtung.

Das System ist insbesondere vorgesehen und ausgebildet, um die Handhabung autoklavierbarer medizinischer Instrumente unmittelbar vor und/oder nach dem Autoklavieren zu vereinfachen. Die Handhabung wird insbesondere vereinfacht, indem einem Inventar ähnliche Daten erzeugt, erfasst oder aktualisiert werden.

Die Kamera kann für das Erfassen von Licht im infraroten Spektralbereich und/oder im für das gesunde menschliche Auge sichtbaren Spektralbereich und/oder im ultravioletten Spektralbereich vorgesehen sein. Die Kamera kann ein monochromatisches oder ein mehrfarbiges Bild erfassen und ein entsprechendes Bildsignal erzeugen. Ferner kann die Kamera für eine monokulare Bilderfassung oder für eine stereoskopische Bilderfassung vorgesehen und ausgebildet sein. Die Kamera kann ein analoges oder ein digitales Bildsignal erzeugen.

Die Bildauswertungseinrichtung kann mit der Kamera in ein Gehäuse integriert sein oder von dieser separat ausgebildet sein. Insbesondere kann der Bildsensor der Kamera mit der Bildauswertungseinrichtung auf einem Halbleiterbauteil (Die) oder in einem elektronischen Bauteil integriert sein. Alternativ kann die Bildauswertungseinrichtung als Computer mit geeigneter Software ausgebildet sein. Das analoge oder digitale Bildsignal kann elektrisch, optisch oder auf andere Weise zu der Bildauswertungseinrichtung übertragen werden.

Die Bildauswertungseinrichtung ist insbesondere vorgesehen und ausgebildet, um ein medizinisches Instrument anhand optisch erkennbarer Merkmale wie der Größe, der Gestalt und/oder Farben zu identifizieren. Dazu umfasst die Bildauswertungseinrichtung insbesondere eine Datenbank, in der entsprechende Merkmale abgelegt und einzelnen Typen medizinischer Instrumente oder einzelnen individuellen medizinischen Instrumenten zugeordnet sind.

Alternativ oder zusätzlich kann die Bildauswertungseinrichtung vorgesehen und ausgebildet sein, um einen Code an einem medizinischen Instrument zu erfassen, beispielsweise einen Barcode, einen QR-Code oder einen anderen ein- oder zweidimensionalen monochromatischen oder polychromatischen Code in Form von Streifen, Punkten, Rechtecken oder alphanumerischen Zeichen. Ein solcher individueller Code kann die Unterscheidung baugleicher medizinischer Instrumente und damit auch für jedes individuelle medizinische Instrument beispielsweise die Erfassung einer exakten Historie ermöglichen oder vereinfachen. Entsprechend umfassen die von der Bildauswertungseinrichtung erzeugten Identifikationsdaten entweder nur den Typ oder die Bauart des medizinischen Instruments oder beispielsweise auch eine Losnummer oder eine Seriennummer des medizinischen Instruments.

Die Datenübertragungseinrichtung umfasst insbesondere einen Sender zum Senden der Identifikationsdaten oder anderer Daten an die Datenempfangseinrichtung der mit dem autoklavierbaren Instrumententräger dauerhaft verbundenen Dateneinrichtung. Der Sender kann zum Senden der Daten in einem analogen oder digitalen Format vorgesehen und ausgebildet sein. Der Sender kann zur überwiegend induktiven oder überwiegend kapazitiven oder zur elektromagnetischen Übertragung von Daten vorgesehen und ausgebildet sein.

Die Datenübertragungseinrichtung kann ferner zum Empfangen oder Auslesen von Identifikationsdaten oder anderen Daten von einer mit einem Instrumententräger dauerhaft verbundenen Datenempfangseinrichtung vorgesehen und ausgebildet sein.

Die Datenübertragungseinrichtung entspricht beispielsweise dem NFC-Standard (near field communication, zu Deutsch: Nahfeldkommunikation ) oder einem anderen RFID-Standard (RFID = radio-frequency identification, im Deutschen: Identifizierung mit Hilfe elektromagnetischer Wellen).

Die Dateneinrichtung ist insbesondere insofern dauerhaft mit dem Instrumententräger mechanisch verbunden, als sie durch die bei der vorgesehenen Verwendung des Instrumententrägers auftretenden statisch oder dynamisch erzeugten Kräfte nicht von dem Instrumententräger getrennt wird. Beispielsweise ist die Dateneinrichtung durch eine Rast- oder Klemmverbindung mit dem Instrumententräger mechanisch verbunden.

Die Dateneinrichtung kann ferner insofern dauerhaft mit dem Instrumententräger mechanisch verbunden sein, als sie nicht ohne Verwendung von Werkzeug zerstörungsfrei von dem Instrumententräger getrennt werden kann. Beispielsweise ist die Dateneinrichtung durch eine Schraub- oder Nietverbindung mit dem Instrumententräger mechanisch verbunden.

Viele medizinische Instrumente sind zu klein, um selbst beispielsweise ein RFID-Etikett aufzunehmen. Für viele kostengünstige medizinische Instrumente ist dies auch nicht wirtschaftlich sinnvoll. Ferner ist das Auslesen vieler RFID-Etiketten auf engem Raum schwierig. Das Auslesen wird ferner durch die in der Regel metallische Struktur des Instrumententrägers erschwert, die als faradayscher Käfig zumindest elektrische und elektromagnetische Felder sehr gut abschirmt.

Das automatisierte optische Identifizieren und gemeinsame Ablegen der Identifikationsdaten in einem mit dem Instrumententräger dauerhaft verbundenen Speicher kann diese Probleme lösen. Das einzelne medizinische Instrument muss kein RFID-Etikett aufweisen, es muss keine Vielzahl von RFID-Etiketten auf engem Raum ausgelesen werden. Dennoch kann der mit dem Instrumententräger dauerhaft verbundene Speicher berührungslos beschrieben und ausgelesen werden.

Ein System, wie es hier beschrieben ist, umfasst insbesondere ferner einem autoklavierbaren Instrumententräger zum Bevorraten, Transportieren und

Bereithalten medizinischer Instrumente und eine mit dem autoklavierbaren Instrumententräger dauerhaft verbundene und autoklavierbare Dateneinrichtung mit einer Datenempfangseinrichtung zum Empfangen von Daten und einem wiederholt beschreibbaren und auslesbaren Speicher zum Speichern von durch die Datenempfangseinrichtung empfangener Daten.

Der Instrumententräger ist insbesondere als Tablett mit einem rechteckigen ebenen Bereich und einem umlaufenden Rand ausgebildet. Der Instrumententräger kann - von der autoklavierbaren Dateneinrichtung abgesehen - einem Standard entsprechen, um mit herkömmlichen Instrumententrägern austauschbar zu sein, diese zu ersetzen und mit diesen zusammen oder anstelle dieser in herkömmlichen Autoklaven autoklaviert zu werden. Der Instrumententräger ist insbesondere aus einem Metallgeflecht oder einem perforierten Metallblech gebildet und wird deshalb auch als Sieb oder Instrumentensieb bezeichnet. Der Instrumententräger kann zur gleichzeitigen Aufnahme eines oder mehrerer medizinischer Instrumente vorgesehen und ausgebildet sein.

Die Dateneinrichtung ist insbesondere ein RFID-Etikett. Die Datenempfangseinrichtung entspricht insbesondere einem RFID-Standard zur überwiegend kapazitiven, überwiegend induktiven oder überwiegend elektromagnetischen Kommunikation. Alternativ oder zusätzlich kann die Datenempfangseinrichtung zum optischen Empfangen von Daten vorgesehen und ausgebildet sein. Die Datenempfangseinrichtung ist insbesondere auch zum Senden von in dem Speicher der Dateneinrichtung gespeicherten Daten vorgesehen und ausgebildet. Die Dateneinrichtung ist insbesondere ausgebildet, um nach Empfang eines Anforderungssignals Daten aus dem Speicher der Dateneinrichtung auszulesen und an den Absender des Anforderungssignals zu senden.

Der Speicher ist insbesondere als nicht-flüchtiger Speicher ausgebildet, der auch ohne Leistungsversorgung Daten speichert. In diesem Fall muss die Dateneinrichtung keinen Energiespeicher zur ständigen Leistungsversorgung des Speichers aufweisen.

Die mit dem Instrumententräger dauerhaft verbundene Dateneinrichtung ermöglicht eine Speicherung von Identitätsdaten von medizinischen Instrumenten, die von dem Instrumententräger aufgenommen sind, unmittelbar und dauerhaft an dem Instrumententräger. Der Speicher der Dateneinrichtung kann jederzeit mit geringem technischem Aufwand und lediglich lokal vorhandener Infrastruktur - beispielsweise einer RFID-Leseeinrichtung - ausgelesen werden, um die Identitätsdaten der medizinischen Instrumente in oder auf dem Instrumententräger zu erhalten. Eine Datenverbindung zu einer anderen Datenbank - beispielsweise auf einem Server eines Krankenhauses - ist nicht erforderlich.

Ein System, wie es hier beschrieben ist, umfasst insbesondere ferner eine mit dem autoklavierbaren Instrumententräger dauerhaft verbundene und autoklavierbare Energieempfangseinrichtung zum Empfangen von Leistung in Form von Licht und zum Bereitstellen elektrischer Leistung für die Dateneinrichtung (22).

Ein System, wie es hier beschrieben ist, umfasst insbesondere ferner eine Lichtquelle zum Bereitstellen von Leistung in Form von Licht für die mit dem autoklavierbaren Instrumententräger dauerhaft verbundene Leistungsem pfangsei nrichtung.

Die Lichtquelle ist insbesondere eine monochromatische oder ein schmales Spektrum emittierende und gerichtete Lichtquelle, beispielsweise ein Laser. Die Lichtquelle ist insbesondere so angeordnet und ausgerichtet, dass sie nur oder im Wesentlichen nur die Leistungsempfangseinrichtung bestrahlt, wenn der autoklavierbare Instrumententräger in der vorgesehenen Weise angeordnet und orientiert ist. Das System kann eine Steuerung aufweisen, die die Lichtquelle nur dann aktiviert, wenn die Dateneinrichtung elektrische Leistung benötigt, insbesondere Daten empfängt, in den Speicher schreibt, liest oder sendet. Das System kann eine Steuerung aufweisen, die die Lichtquelle auf die Leistungsempfangseinrichtung richtet.

Das Spektrum der Lichtquelle ist insbesondere auf die spektrale Empfindlichkeit der Leistungsempfangseinrichtung abgestimmt, so das beide Spektren einen möglichst großen Überlapp aufweisen. Leistung kann durch Licht im infraroten Spektralbereich und/oder im für das gesunde menschliche Auge sichtbaren Spektralbereich und/oder im ultravioletten Spektralbereich übertragen werden.

Die Leistungsempfangseinrichtung ist insbesondere in die Dateneinrichtung integriert und bildet mit dieser eine autoklavierbare Einheit.

Aufgrund der Leistungsversorgung durch die Lichtquelle und die Leistungsempfangseinrichtung ist kein Energiespeicher in der Dateneinrichtung erforderlich. Dies kann eine Miniaturisierung und geringere Herstellungskosten sowie eine höhere Temperaturfestigkeit der Dateneinrichtung ermöglichen.

Ein System, wie es hier beschrieben ist, umfasst insbesondere ferner eine Projektionseinrichtung zum sichtbaren Projizieren einer vorgesehenen Soll-Position eines medizinischen Instruments auf eine Oberfläche des autoklavierbaren Instrumententrägers.

Die Projektionseinrichtung ist insbesondere dafür vorgesehen und ausgebildet, einen Umriss eines medizinischen Instruments in der für das medizinische Instrument vorgesehenen Anordnung und Orientierung auf den Instrumententräger zu projizieren. Beispielsweise kann ein Laserestrahl den Umriss des medizinischen Instruments beschreiben.

Die Projektion der vorgesehenen Position und optional auch Orientierung des medizinischen Instruments auf den Instrumententräger kann die manuelle Bestückung des Instrumententrägers vereinfachen und zuverlässiger machen.

Ein System, wie es hier beschrieben ist, umfasst insbesondere ferner eine Datenbank, in der vorgesehene Positionen medizinischer Instrumente auf einer Oberfläche des autoklavierbaren Instrumententrägers gespeichert sind.

In der Datenbank sind insbesondere für verschiedene medizinische Maßnahmen verschiedene jeweils an die Maßnahme angepasste Bestückungen der autoklavierbaren Instrumententräger abgelegt. Zu jeder Bestückung können vorbestimmte Orte und Orientierungen für alle enthaltenen medizinischen Instrumente gespeichert sein.

Ein autoklavierbarer Instrumententräger zum Bevorraten, Transportieren und Bereithalten medizinischer Instrumente umfasst eine mit dem autoklavierbaren Instrumententräger dauerhaft verbundene und autoklavierbare Dateneinrichtung mit einem wiederholt beschreibbaren und auslesbaren Speicher und einer Datenübertragungseinrichtung zum Empfangen und Senden von Daten und eine mit dem autoklavierbaren Instrumententräger dauerhaft verbundene und autoklavierbare Energieempfangseinrichtung zum Empfangen von Leistung in Form von Licht und zum Bereitstellen elektrischer Leistung für die Dateneinrichtung.

Der autoklavierbare Instrumententräger ist insbesondere vorgesehen, um Teil eines Systems, wie es hier beschrieben ist, zur Unterstützung der Handhabung medizinischer Instrumente zu sein. Der autoklavierbare Instrumententräger weist insbesondere Merkmale, Eigenschaften und Funktionen auf, wie sie hier in Zusammenhang mit dem autoklavierbaren Instrumententräger des beschriebenen Systems beschrieben sind.

Der Instrumententräger ist insbesondere als Tablett mit einem rechteckigen ebenen Bereich und einem umlaufenden Rand ausgebildet. Der Instrumententräger kann - von der autoklavierbaren Dateneinrichtung abgesehen - einem Standard entsprechen, um mit herkömmlichen Instrumententrägern austauschbar zu sein, diese zu ersetzen und mit diesen zusammen oder anstelle dieser in herkömmlichen Autoklaven autoklaviert zu werden. Der Instrumententräger ist insbesondere aus einem Metallgeflecht oder einem perforierten Metallblech gebildet und wird deshalb auch als Sieb oder Instrumentensieb bezeichnet. Der Instrumententräger kann zur gleichzeitigen Aufnahme eines oder mehrerer medizinischer Instrumente vorgesehen und ausgebildet sein.

Die Dateneinrichtung ist insbesondere ein RFID-Etikett. Die Datenempfangseinrichtung entspricht insbesondere einem RFID-Standard zur überwiegend kapazitiven, überwiegend induktiven oder überwiegend elektromagnetischen Kommunikation. Alternativ oder zusätzlich kann die Datenempfangseinrichtung zum optischen Empfangen von Daten vorgesehen und ausgebildet sein. Die Datenempfangseinrichtung ist insbesondere auch zum Senden von in dem Speicher der Dateneinrichtung gespeicherten Daten vorgesehen und ausgebildet. Die Dateneinrichtung ist insbesondere ausgebildet, um nach Empfang eines Anforderungssignals Daten aus dem Speicher der Dateneinrichtung auszulesen und an den Absender des Anforderungssignals zu senden.

Die Dateneinrichtung ist insbesondere insofern dauerhaft mit dem Instrumententräger mechanisch verbunden, als sie durch die bei der vorgesehenen Verwendung des Instrumententrägers auftretenden statisch oder dynamisch erzeugten Kräfte nicht von dem Instrumententräger getrennt wird. Beispielsweise ist die Dateneinrichtung durch eine Rast- oder Klemmverbindung mit dem Instrumententräger mechanisch verbunden.

Die Dateneinrichtung kann ferner insofern dauerhaft mit dem Instrumententräger mechanisch verbunden sein, als sie nicht ohne Verwendung von Werkzeug zerstörungsfrei von dem Instrumententräger getrennt werden kann. Beispielsweise ist die Dateneinrichtung durch eine Schraub- oder Nietverbindung mit dem Instrumententräger mechanisch verbunden.

Der Speicher ist insbesondere als nicht-flüchtiger Speicher ausgebildet, der auch ohne Leistungsversorgung Daten speichert. In diesem Fall muss die Dateneinrichtung keinen Energiespeicher zur ständigen Leistungsversorgung des Speichers aufweisen.

Die Leistungsempfangseinrichtung kann zum Empfangen von Leistung in Form von Licht im infraroten Spektralbereich, im für das gesunde menschliche Auge sichtbaren Spektralbereich und/oder im ultravioletten Spektralbereich vorgesehen und ausgebildet sein. Die Leistungsempfangseinrichtung ist insbesondere in die Dateneinrichtung integriert und bildet mit dieser eine autoklavierbare Einheit.

Die mit dem Instrumententräger dauerhaft verbundene Dateneinrichtung ermöglicht eine Speicherung von Identitätsdaten von medizinischen Instrumenten, die von dem Instrumententräger aufgenommen sind, unmittelbar und dauerhaft an dem Instrumententräger. Der Speicher der Dateneinrichtung kann jederzeit mit geringem technischem Aufwand und lediglich lokal vorhandener Infrastruktur - beispielsweise einer RFID-Leseeinrichtung - ausgelesen werden, um die Identitätsdaten der medizinischen Instrumente in oder auf dem Instrumententräger zu erhalten. Eine Datenverbindung zu einer anderen Datenbank - beispielsweise auf einem Server eines Krankenhauses - ist nicht erforderlich.

Aufgrund der Leistungsversorgung durch die Lichtquelle und die Leistungsempfangseinrichtung ist kein Energiespeicher in der Dateneinrichtung erforderlich. Dies kann eine Miniaturisierung und geringere Herstellungskosten sowie eine höhere Temperaturfestigkeit der Dateneinrichtung ermöglichen.

Alternativ oder zusätzlich können die Identitätsdaten der medizinischen Instrumente über eine Datenverbindung zu einer separaten Datenbank übertragen und dort gespeichert und/oder von einer separaten Datenbank gelesen werden. Ein Speichern von Identitätsdaten sowohl in der Dateneinrichtung an dem autoklavierbaren Instrumententräger als auch in einer separaten Datenbank kann das Risiko eines Verlusts von Identitätsdaten deutlich mindern. Durch einen Vergleich von in der Dateneinrichtung an dem autoklavierbaren Instrumententräger gespeicherten Identitätsdaten und in einer separaten Datenbank gespeicherten Identitätsdaten kann die Integrität der Identitätsdaten bestätigt werden. Auch wenn vorübergehend keine Identitätsdaten aus der Dateneinrichtung an dem autoklavierbaren Instrumententräger ausgelesen werden können oder diese dauerhaft verloren sind, können sie mit hoher Wahrscheinlichkeit aus der separaten Datenbank gelesen werden, und umgekehrt.

Ein autoklavierbarer Instrumententräger, wie er hier beschrieben ist, umfasst insbesondere ferner einen Barcode oder einen anderen ein- oder zweidimensionalen optisch lesbaren Code oder eine andere optisch lesbare Einrichtung zur Identifizierung des autoklavierbaren Instrumententrägers.

Ein autoklavierbarer Instrumententräger zum Bevorraten, Transportieren und Bereithalten medizinischer Instrumente umfasst eine mit dem autoklavierbaren Instrumententräger dauerhaft verbundene und autoklavierbare Dateneinrichtung mit einem wiederholt beschreibbaren und auslesbaren Speicher und einer Datenübertragungseinrichtung zum Senden und Empfangen von Daten und einen Barcode oder einen anderen ein- oder zweidimensionalen optisch lesbaren Code oder eine andere optisch lesbaren Einrichtung zur Identifizierung des autoklavierbaren Instrumententrägers.

Der an dem autoklavierbaren Instrumententräger angebrachte Barcode oder andere ein- oder zweidimensionale optisch lesbare Code oder die an dem autoklavierbaren Instrumententräger angebrachten andere optisch lesbare Einrichtung schafft eine weitere Möglichkeit, den autoklavierbaren Instrumententräger zu identifizieren. Wenn die Identität des autoklavierbaren Instrumententrägers bekannt ist, kann aus einer Datenbank der Inhalt oder die Bestückung des autoklavierbaren Instrumententrägers gelesen werden. Ferner kann der optisch lesbare Code oder die optisch lesbare Einrichtung eine Verifikation ermöglichen, um auszuschließen, dass beispielsweise mittels einer RFID-Leseeinrichtung irrtümlich eine Dateneinrichtung an einem anderen Instrumententräger ausgelesen wurde.

Ein autoklavierbarer Sterilbehälter zum Bevorraten, Transportieren und Bereithalten eines oder mehrerer Instrumententräger mit jeweils einem oder mehreren medizinischen Instrumenten umfasst eine mit dem autoklavierbaren Sterilbehälter dauerhaft verbundene und autoklavierbare Dateneinrichtung mit einem wiederholt beschreibbaren und auslesbaren Speicher und einer Datenübertragungseinrichtung zum Senden und Empfangen von Daten und eine mit dem autoklavierbaren Sterilbehälter dauerhaft verbundene und autoklavierbare Energieempfangseinrichtung zum Empfangen von Leistung in Form von Licht und zum Bereitstellen elektrischer Leistung für die Dateneinrichtung.

Der autoklavierbare Sterilbehälter ist insbesondere zur Aufnahme von einem oder mehreren autoklavierbaren Instrumententrägern, wie sie hier beschrieben sind, vorgesehen und ausgebildet. Der autoklavierbare Sterilbehälter ist insbesondere zur Verwendung mit einem System, wie es hier beschrieben ist, vorgesehen und ausgebildet. Sterilbehälter werden oft auch als Sterilcontainer bezeichnet.

Der autoklavierbare Sterilbehälter weist ist insbesondere einen Deckel oder eine andere Einrichtung zum Verschließen auf. Im verschlossenen Zustand verhindert der autoklavierbare Sterilbehälter insbesondere das Eindringen von Keimen oder anderen Partikeln und optional auch von Flüssigkeiten oder Gasen in den autoklavierbaren Sterilbehälter. Auf diese Weise bleibt in dem autoklavierbaren Sterilbehälter enthaltenes Sterilgut steril bis der Sterilbehälter wieder geöffnet wird.

Der autoklavierbare Sterilbehälter kann eine Membran aufweisen, die während des Autoklavierprozesses ein Eindringen von heißem Wasserdampf zur Sterilisation der medizinischen Instrumente auf den Instrumententrägern, der Instrumententräger und der inneren Oberflächen des Sterilbehälters ermöglicht, aber das Eindringen von Keimen oder anderen Partikeln verhindert. Die Membran kann an einem Deckel oder einer anderen Einrichtung zum Verschließen vorgesehen sein oder den Deckel oder die andere Einrichtung zum Verschließen ganz oder teilweise bilden.

Im Übrigen kann der autoklavierbare Sterilbehälter ähnliche Merkmale, Eigenschaften und Funktionen aufweisen wie ein autoklavierbarer Instrumententräger wie er hier beschrieben ist.

Die Dateneinrichtung ist insbesondere ganz oder teilweise an einer Außenseite des Sterilbehälters angeordnet, so dass sie von außerhalb beschrieben oder ausgelesen werden kann. Die Dateneinrichtung ist insbesondere ein RFID-Etikett. Die Datenempfangseinrichtung entspricht insbesondere einem RFID-Standard zur überwiegend kapazitiven, überwiegend induktiven oder überwiegend elektromagnetischen Kommunikation. Alternativ oder zusätzlich kann die Datenempfangseinrichtung zum optischen Empfangen von Daten vorgesehen und ausgebildet sein. Die Datenempfangseinrichtung ist insbesondere auch zum Senden von in dem Speicher der Dateneinrichtung gespeicherten Daten vorgesehen und ausgebildet. Die Dateneinrichtung ist insbesondere ausgebildet, um nach Empfang eines Anforderungssignals Daten aus dem Speicher der Dateneinrichtung auszulesen und an den Absender des Anforderungssignals zu senden.

Die Dateneinrichtung ist insbesondere insofern dauerhaft mit dem Sterilbehälter mechanisch verbunden, als sie durch die bei der vorgesehenen Verwendung des Sterilbehälters auftretenden statisch oder dynamisch erzeugten Kräfte nicht von dem Sterilbehälter getrennt wird. Beispielsweise ist die Dateneinrichtung durch eine Rast- oder Klemmverbindung mit dem Sterilbehälter mechanisch verbunden.

Die Dateneinrichtung kann ferner insofern dauerhaft mit dem Sterilbehälter mechanisch verbunden sein, als sie nicht ohne Verwendung von Werkzeug zerstörungsfrei von dem Sterilbehälter getrennt werden kann. Beispielsweise ist die Dateneinrichtung durch eine Schraub- oder Nietverbindung mit dem Sterilbehälter mechanisch verbunden.

Der Speicher ist insbesondere als nicht-flüchtiger Speicher ausgebildet, der auch ohne Leistungsversorgung Daten speichert. In diesem Fall muss die Dateneinrichtung keinen Energiespeicher zur ständigen Leistungsversorgung des Speichers aufweisen.

Die Leistungsempfangseinrichtung kann zum Empfangen von Leistung in Form von Licht im infraroten Spektralbereich, im für das gesunde menschliche Auge sichtbaren Spektralbereich und/oder im ultravioletten Spektralbereich vorgesehen und ausgebildet sein. Die Leistungsempfangseinrichtung ist insbesondere in die Dateneinrichtung integriert und bildet mit dieser eine autoklavierbare Einheit.

Die mit dem Sterilbehälter dauerhaft verbundene Dateneinrichtung ermöglicht eine Speicherung von Identitätsdaten von medizinischen Instrumenten, die in dem Sterilbehälter aufgenommen sind, unmittelbar und dauerhaft an dem Sterilbehälter. Der Speicher der Dateneinrichtung kann jederzeit mit geringem technischem Aufwand und lediglich lokal vorhandener Infrastruktur - beispielsweise einer RFID-Leseeinrichtung - ausgelesen werden, um die Identitätsdaten der medizinischen Instrumente in dem Sterilbehälter zu erhalten. Eine Datenverbindung zu einer anderen Datenbank - beispielsweise auf einem Server eines Krankenhauses - ist nicht erforderlich.

Aufgrund der Leistungsversorgung durch die Lichtquelle und die Leistungsempfangseinrichtung ist kein Energiespeicher in der Dateneinrichtung erforderlich. Dies kann eine Miniaturisierung und geringere Herstellungskosten sowie eine höhere Temperaturfestigkeit der Dateneinrichtung ermöglichen.

Ein autoklavierbarer Sterilbehälter wie er hier beschrieben ist, umfasst insbesondere ferner einem Barcode oder einem anderen ein- oder zweidimensionalen optisch lesbaren Code oder einer anderen optisch lesbaren Einrichtung zur Identifizierung des autoklavierbaren Sterilbehälters.

Ein autoklavierbarer Sterilbehälter zum Bevorraten, Transportieren und Bereithalten eines oder mehrerer Instrumententräger mit jeweils einem oder mehreren medizinischen Instrumenten umfasst eine mit dem autoklavierbaren Sterilbehälter dauerhaft verbundene und autoklavierbaren Dateneinrichtung mit einem wiederholt beschreibbaren und auslesbaren Speicher und einer Datenübertragungseinrichtung zum Senden und Empfangen von Daten und einen Barcode oder einen anderen ein- oder zweidimensionalen optisch lesbaren Code oder einer anderen optisch lesbaren Einrichtung zur Identifizierung des autoklavierbaren Sterilbehälters.

Der an dem autoklavierbaren Instrumententräger angebrachte Barcode oder andere ein- oder zweidimensionale optisch lesbare Code oder die an dem autoklavierbaren Instrumententräger angebrachten andere optisch lesbare Einrichtung schafft eine weitere Möglichkeit, den autoklavierbaren Instrumententräger zu identifizieren. Wenn die Identität des autoklavierbaren Instrumententrägers bekannt ist, kann aus einer Datenbank der Inhalt oder die Bestückung des autoklavierbaren Instrumententrägers gelesen werden. Ferner kann der optisch lesbare Code oder die optisch lesbare Einrichtung eine Verifikation ermöglichen, um auszuschließen, dass beispielsweise mittels einer RFID-Leseeinrichtung irrtümlich eine Dateneinrichtung an einem anderen Instrumententräger ausgelesen wurde.

Bei einem autoklavierbaren Instrumententräger, wie er hier beschrieben ist, oder einem autoklavierbaren Sterilbehälter, wie er hier beschrieben ist, umfasst die Energieempfangseinrichtung insbesondere eine photovoltaische Zelle oder einen optoelektrischen Wandler auf der Basis von Galliumarsenid.

Ein Verfahren zur Unterstützung der Handhabung medizinischer Instrumente umfasst ein
optisches Erfassen eines Bilds eines oder mehrerer medizinischer Instrumente, ein Erzeugen eines Bildsignals, das das erfasste Bild repräsentiert, ein Übertragen des Bildsignals zu einer Bildauswertungseinrichtung, ein Auswerten des Bildsignals durch die Bildauswertungseinrichtung, um das oder die in dem Bild abgebildeten medizinischen Instrumente zu identifizieren, ein Erzeugen von Identitätsdaten, die die Identitäten des oder der identifizierten medizinischen Instrumente repräsentieren, ein Übertragen von Identitätsdaten und ein Speichern der Identitätsdaten in einem mit einem Instrumententräger dauerhaft verbundenen Speicher oder ein Vergleichen der Identitätsdaten mit Identitätsdaten, die in dem mit dem Instrumententräger dauerhaft verbundenen Speicher gespeichert sind.

Das Verfahren ist insbesondere mit einem System, wie es hier beschrieben ist, mit einem autoklavierbaren Instrumententräger, wie er hier beschrieben ist, und/oder mit einem autoklaveribaren Sterilbehälter, wie er hier beschrieben ist, ausführbar.

Das Verfahren kann insbesondere die Handhabung autoklavierbarer medizinischer Instrumente unmittelbar vor und/oder nach dem Autoklavieren vereinfachen. Die Handhabung wird insbesondere vereinfacht, indem einem Inventar ähnliche Daten erzeugt, erfasst oder aktualisiert werden.

Das Bild kann im infraroten Spektralbereich und/oder im für das gesunde menschliche Auge sichtbaren Spektralbereich und/oder im ultravioletten Spektralbereich vorgesehen sein. Das Bild kann monochromatisch oder mehrfarbig erfasst werden und ein entsprechendes Bildsignal erzeugen. Das Bild kann monokular oder stereoskopisch erfasst werden vorgesehen und ausgebildet sein. Das Bildsignal kann analog oder digital sein.

Das medizinische Instrument wird anhand optisch erkennbarer Merkmale wie der Größe, der Gestalt und/oder Farben identifiziert werden. Dazu vergleicht die Bildauswertungseinrichtung Merkmale in dem durch das Bildsignal repräsentierten Bild insbesondere mit entsprechenden Merkmalen, die in einer Datenbank abgelegt und einzelnen Typen medizinischer Instrumente oder einzelnen individuellen medizinischen Instrumenten zugeordnet sind.

Alternativ oder zusätzlich kann ein Code an einem medizinischen Instrument erfasst werden, beispielsweise einen Barcode, einen QR-Code oder ein anderer ein- oder zweidimensionaler monochromatischer oder polychromatischer Code in Form von Streifen, Punkten, Rechtecken oder alphanumerischen Zeichen. Ein solcher individueller Code kann die Unterscheidung baugleicher medizinischer Instrumente und damit auch für jedes individuelle medizinische Instrument beispielsweise die Erfassung einer exakten Historie ermöglichen oder vereinfachen. Entsprechend umfassen die erzeugten Identifikationsdaten entweder nur den Typ oder die Bauart des medizinischen Instruments oder beispielsweise auch eine Losnummer oder eine Seriennummer des medizinischen Instruments.

Die Übertragung der Identitätsdaten erfolgt insbesondere entsprechend dem NFC-Standard (near field communication, zu Deutsch: Nahfeldkommunikation ) oder einem anderen RFID-Standard (RFID = radio-frequency identification, im Deutschen: Identifizierung mit Hilfe elektromagnetischer Wellen).

Insbesondere werden die erzeugten Identitätsdaten zu dem mit dem Instrumententräger dauerhaft verbundenen Speicher übertragen und dort gespeichert. Alternativ werden in dem mit dem Instrumententräger dauerhaft verbundenen Speicher gespeicherte Identitätsdaten aus diesem ausgelesen, zu einer Einrichtung zum Vergleichen übertragen und dort mit den erzeugten Identitätsdaten verglichen.

Viele medizinische Instrumente sind zu klein, um selbst beispielsweise ein RFID-Etikett aufzunehmen. Für viele kostengünstige medizinische Instrumente ist dies auch nicht wirtschaftlich sinnvoll. Ferner ist das Auslesen vieler RFID-Etiketten auf engem Raum schwierig. Das Auslesen wird ferner durch die in der Regel metallische Struktur des Instrumententrägers und weiterer medizinischer Instrumente darin erschwert, die als faradayscher Käfig wirken und zumindest elektrische und elektromagnetische Felder sehr gut abschirmt.

Das automatisierte optische Identifizieren und gemeinsame Ablegen der Identifikationsdaten in einem mit dem Instrumententräger dauerhaft verbundenen Speicher oder das Auslesen von Identifikationsdaten aus diesem für einen nachfolgenden Vergleich kann diese Probleme lösen. Das einzelne medizinische Instrument muss kein RFID-Etikett aufweisen, es muss keine Vielzahl von RFID-Etiketten auf engem Raum ausgelesen werden. Dennoch kann der mit dem Instrumententräger dauerhaft verbundene Speicher berührungslos beschrieben und ausgelesen werden.

Bei einem Verfahren, wie es hier beschrieben ist, umfasst das Auswerten des Bildsignals durch die Bildauswertungseinrichtung insbesondere ein Erfassen der Anordnung der in dem Bild abgebildeten medizinischen Instrumente, wobei die erfasste Anordnung als Soll-Anordnung der medizinischen Instrumente (10) gespeichert wird.

Erfasst wird insbesondere die Anordnung eines oder mehrerer medizinischer Instrumente auf einem Instrumententräger, wobei die Anordnung optional die Orientierung jedes medizinischen Instruments umfasst. Das Speichern der erfassten Anordnung, optional umfassend die erfasste Orientierung, als Soll-Anordnung kann Teil einer Lern-Phase oder Trainingsphase für ein System sein. In dieser Lern-Phase oder Trainingsphase wird die Soll-Anordnung, optional umfassend die Soll-Orientierung, insbesondere manuell erzeugt, dann als Referenz für jede folgende Bestückung eines Instrumententrägers erfasst und später als Referenz verwendet.

Die Soll-Anordnung kann in einem mit dem Instrumententräger dauerhaft verbundenen Speicher oder in einer Datenbank an einem anderen Ort gespeichert werden.

Ein Verfahren, wie es hier beschrieben ist, umfasst insbesondere ferner ein Ermitteln einer Soll-Anordnung eines identifizierten medizinischen Instruments auf einem Instrumententräger und ein Projizieren des medizinischen Instruments in der Soll-Anordnung auf den Instrumententräger.

Das Ermitteln der Soll-Anordnung, optional umfassend eine Soll-Orientierung, umfasst insbesondere das Auswählen von einer von mehreren gespeicherten Soll-Anordnungen, und zwar derjenigen Soll-Anordnung die dem identifizierten medizinischen Instrument zugeordnet ist. Das Ermitteln der Soll-Anordnung umfasst ferner das Lesen der ausgewählten Soll-Anordnung. Die Soll-Anordnung kann aus einem mit dem Instrumententräger dauerhaft mechanisch verbundenen Speicher oder aus beispielsweise aus einer separaten Datenbank gelesen werden.

Das Projizieren des medizinischen Instruments in der Soll-Anordnung umfasst insbesondere ein Projizieren des Umrisses des medizinischen Instruments in der Soll-Anordnung auf den Instrumententräger. Der Umriss des medizinischen Instruments wird beispielsweise von einem gelenkten Laserstrahl auf dem Instrumententräger schnell wiederholt abgefahren, so dass ein im Rahmen der Wahrnehmung durch das menschliche Auge unbewegtes kontinuierliches Bild erzeugt wird. Alternativ kann der Umriss oder auch ein detailliertes Bild des identifizierten medizinischen Instruments auf den Instrumententräger projiziert werden.

Ein Verfahren, wie es hier beschrieben ist, umfasst insbesondere ferner ein optisches Erfassen eines weiteren Bilds des oder der medizinischen Instrumente auf dem Instrumententräger, ein Erzeugen eines weiteren Bildsignals, das das erfasste weitere Bild repräsentiert, ein Übertragen des weiteren Bildsignals zu der Bildauswertungseinrichtung, ein Auswerten des weiteren Bildsignals durch die Bildauswertungseinrichtung, um die Anordnung des oder der in dem weiteren Bild abgebildeten medizinischen Instrumente zu bestimmen, und ein Erzeugen eines Meldesignals abhängig davon, ob die bestimmte Anordnung des oder der medizinischen Instrumente ihrer Soll-Anordnung entspricht.

Das weitere Bild wird insbesondere erfasst, nachdem die Soll-Anordnung eines identifizierten medizinischen Instruments ermittelt und auf den Instrumententräger projiziert und das medizinische Instrument auf dem Instrumententräger abgelegt wurde. Die folgenden Verfahrensschritte dienen der Verifikation der korrekten Anordnung des medizinischen Instruments und der Rückmeldung an eine Person, die den Instrumententräger bestückt. Alternativ kann das weitere Bild beispielsweise bei einer automatisierten Bestückung eines Instrumententrägers durch einen Roboterarm erfasst werden, um zu verifizieren, ob das medizinische Instrument korrekt abgelegt wurde, also entsprechend der Soll-Anordnung angeordnet und optional auch orientiert ist.

Das Meldesignal ist beispielsweise ein akustisches oder optisches Signal für eine den Instrumententräger bestückende Person. Alternativ kann das Meldesignal ein elektrisch oder optisch oder auf andere Weise übertragenes Signal sein, das ein Fortsetzen der Bestückung auslöst oder zulässt oder eine Korrektur der Anordnung des medizinischen Instruments auslöst.

Bei einem Verfahren, wie es hier beschrieben ist, ist das Erzeugen des Meldesignals insbesondere ferner abhängig davon, ob das Speichern der Identitätsdaten des oder der identifizierten medizinischen Instrumente in dem mit dem Instrumententräger dauerhaft verbundenen Speicher erfolgreich war.

Ein Verfahren, wie es hier beschrieben ist, umfasst insbesondere ferner ein Setzen des Attributs "unsteril" zu einem medizinischen Instrument, wenn der Vergleich der Identitätsdaten ergibt, dass das medizinische Instrument nicht mehr an dem Instrumententräger angeordnet ist.

Der Vergleich und das Setzen des Attributs erfolgen insbesondere nachdem der Instrumententräger in einen Operationssaal oder an einem anderen Ort einer medizinischen Maßnahme in Gebrauch genommen und dazu beispielsweise einem Sterilcontainer entnommen wurde.

Das Attribut "unsteril" wird insbesondere in dem mit dem Instrumententräger dauerhaft verbundenen Speicher gesetzt. Alternativ oder zusätzlich wird das Attribut "unsteril" in einem anderen Speicher gesetzt, beispielsweise in einer zentralen Datenbank, in der alle medizinischen Instrumente einer medizinischen Einrichtung inventarisiert sind. Gleichzeitig kann ein Zähler inkrementiert werden, der die Zahl der Verwendungen des medizinischen Instruments zählt.

Ein Verfahren, wie es hier beschrieben ist, umfasst insbesondere ferner ein Einbringen des Instrumententrägers in einen Sterilbehälter, ein Auslesen der Identitätsdaten des oder der identifizierten medizinischen Instrumente aus dem mit dem Instrumententräger dauerhaft verbundenen Speicher, ein Übertragen der Identitätsdaten des oder der identifizierten medizinischen Instrumente zu einem mit dem Sterilbehälter dauerhaft verbundenen Speicher und ein Speichern der Identitätsdaten des oder der identifizierten medizinischen Instrumente in dem mit dem Sterilbehälter dauerhaft verbundenen Speicher.

Alternativ werden die Identitätsdaten des oder der identifizierten medizinischen Instrumente aus einem anderen Speicher, beispielsweise aus einer Datenbank, ausgelesen, in dem abgelegt ist, welche medizinischen Instrumente in welchem Instrumententräger abgelegt sind.

Das Auslesen der Identitätsdaten des oder der medizinischen Instrumente aus dem mit dem Instrumententräger dauerhaft verbundenen Speicher erfolgt insbesondere entsprechend einem RFID-Standard. Das Übertragen der Identitätsdaten des oder der medizinischen Instrumente erfolgt insbesondere entsprechend einem RFID-Standard.

Das Speichern der Identitätsdaten in einem mit dem Sterilbehälter dauerhaft verbundenen Speicher kann die Identitätsdaten auch dann von außen zugänglich machen, wenn der Sterilbehälter - beispielsweise als faradayscher Käfig wirkend - das Auslesen eines Speichers an einem Instrumententräger innerhalb des Sterilbehälters verhindern würde. Dazu ist der mit dem Sterilbehälter dauerhaft verbundene Speicher insbesondere über eine Sende- und Empfangseinrichtung an einer Außenseite oder einer äußeren Oberfläche des Sterilbehälters auslesbar.

### Kurzbeschreibung der Figuren

Nachfolgend werden Ausführungsformen anhand der beigefügten Figuren näher erläutert. Es zeigen:
- Figur 1: eine schematische Darstellung eines Systems zur Unterstützung der Handhabung medizinischer Instrumente;
- Figur 2: eine schematische Darstellung einer Dateneinrichtung des Systems aus Figur 1;
- Figur 3: eine schematische Darstellung eines weiteren Systems zur Unterstützung der Handhabung medizinischer Instrumente;
- Figur 4: ein schematisches Flussdiagramm eines Verfahrens zur Unterstützung der Handhabung medizinischer Instrumente;
- Figur 5: ein schematisches Flussdiagramm eines Verfahrens zur Unterstützung der Handhabung medizinischer Instrumente;
- Figur 6: ein schematisches Flussdiagramm eines Verfahrens zur Unterstützung der Handhabung medizinischer Instrumente;
- Figur 7: ein schematisches Flussdiagramm eines Verfahrens zur Unterstützung der Handhabung medizinischer Instrumente;
- Figur 8: ein schematisches Flussdiagramm eines Verfahrens zur Unterstützung der Handhabung medizinischer Instrumente.

### Beschreibung der Ausführungsformen

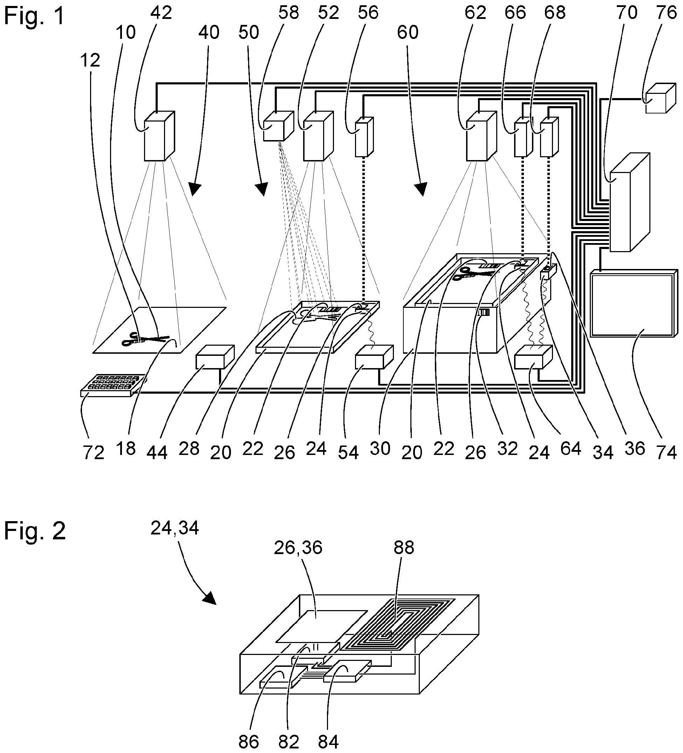

Figur 1 zeigt eine schematische Darstellung eines Systems zur Unterstützung der Handhabung medizinischer Instrumente 10 insbesondere nach der Reinigung, unmittelbar vor oder nach der Sterilisation und vor der Verwendung. In Figur 1 ist ein medizinisches Instrument dargestellt, das einen optisch lesbaren Code 12 aufweist. Das medizinische Instrument kann auf einer Ablagefläche 18 abgelegt werden.

Ein Instrumententräger 20 weist einen optisch lesbaren Code 22, beispielsweise einen Barcode, und ein RFID-Etikett 24 auf. Der optische Code 22 und das RFID-Etikett 24 sind mit dem Instrumententräger 20 insbesonderer dauerhaft mechanisch verbunden, können also zumindest ohne Verwendung von Werkzeug nicht zerstörungsfrei von dem Instrumententräger 20 getrennt werden. Bei dem dargestellten Beispiel sind der optisch lesbare Code 22 an einem geraden Randabschnitt und das RFID-Etikett 24 in einer Ecke des Innenraums mit an sich rechteckiger Grundform angeordnet. Alternativ können der optisch lesbare Code 22 und das RFID-Etikett 24 an anderen Orten in oder an dem Instrumententräger angeordnet sein, beispielsweise in eine Seitenwand des Instrumententrägers 20 integriert.

Bei dem dargestellten Beispiel weist das RFID-Etikett 24 ein photoelektrisches Bauelement 26 zum Empfangen von Leistung in Form von Licht und zum Bereitstellen elektrischer Leistung für elektronische Schaltungen des RFID-Etiketts 24 auf.

Der Instrumententräger 20 ist beispielsweise aus Metallgeflecht oder einem perforierten Metallblech gebildet und wird deshalb auch als Sieb oder Instrumentensieb bezeichnet. Der Instrumententrägers 20 kann einem Industriestandard entsprechen, um herkömliche Instrumententräger ersetzen zu können. Der Instrumententräger 20 ist sterilisierbar und insbesondere autoklavierbar, wird also auch durch wiederholte Einwirkung von reinem Wasserdampf einer Temperatur im Bereich von 140 Grad Celsius und nicht beschädigt oder verändert.

In der dargestellten Situation ist ein Umriss 28 des medizinischen Instruments 10 in einer vorgesehenen Anordnung auf den Instrumententräger projiziert.

Ein Sterilbehälter 30 - auch als Sterilcontainer bezeichnet - weist einen optisch lesbaren Code 32, beispielsweise einen Barcode, und ein RFID-Etikett 34 auf. Der optische Code 32 und das RFID-Etikett 34 sind mit dem Sterilbehälter 30 insbesonderer dauerhaft mechanisch verbunden, können also zumindest ohne Verwendung von Werkzeug nicht zerstörungsfrei von dem Sterilbehälter 30 getrennt werden. Bei dem dargestellten Beispiel sind der optisch lesbare Code 22 und das RFID-Etikett 34 an äußeren Oberflächenbereichen des Sterilbehälters 30 angeordnet. Alternativ können der optisch lesbare Code 32 und das RFID-Etikett 34 an anderen Orten in oder an dem Sterilbehälter angeordnet sein. Beispielsweise kann das RFID-Etikett 34 in eine Seitenwand des Sterilbehälters 30 integriert sein.

Bei dem dargestellten Beispiel weist das RFID-Etikett 34 ein photoelektrisches Bauelement 36 zum Empfangen von Leistung in Form von Licht und zum Bereitstellen elektrischer Leistung für elektronische Schaltungen des RFID-Etiketts 34 auf.

Der Sterilbehälter 30 ist zur Aufnahme eines oder mehrerer Instrumententräger 20 vorgesehen und ausgebildet. Der Sterilbehälter 30 ist insbesondere aus gasdichten Material, beispielsweise einem Metallblech, gebildet und durch einen nicht dargestellten Deckel gasdicht oder zumindest in einer Weise, die das Eindringen von Flüssigkeiten und Partikeln verhindert, verschließbar. Der Sterilbehälter 30 kann einem Industriestandard entsprechen, um herkömmliche Sterilbehälter ersetzen zu können. Der Sterilbehälter 30 ist sterilisierbar und insbesondere autoklavierbar, wird also auch durch wiederholte Einwirkung von reinem Wasserdampf einer Temperatur im Bereich von 140 Grad Celsius und nicht beschädigt oder verändert. Der Sterilbehälter 30 ist dafür vorgesehen, auf die gleiche Weise sterilisierbare medizinische Instrumente 10 und Instrumententräger 20 aufzunehmen, insbesondere während und nach der Sterilisation.

Bei der in Figur 1 gezeigten Situation oder Konfiguration sind in dem Sterilbehälter 30 bereits mehrere Instrumententräger 20 angeordnet, wobei der oberste Instrumententräger 20 sichtbar ist.

Ein erster Bereich 40 zum Identifzieren des medizinischen Instruments 10 umfasst vor allem die Ablagefläche 18, auf der in der dargestellten Situation das medizinische Instrument abgelegt ist. Der erste Bereich 40 und in diesem angeordnete Objekte werden von einer ersten Kamera 42 optisch erfasst. Dabei erfasst die erste Kamera 42 alle auf der Ablagefläche 18 abgelegten medizinischen Instrumente 10. Optional kann die erste Kamera 42 optisch lesbare Codes 12 an medizinischen Instrumenten 10 erfassen.

Nahe der Ablagefläche 18 und dem ersten Bereich 40 ist ferner eine RFID-Leseeinrichtung 44 zum Lesen von RFID-Etiketten, die sich in dem ersten Bereich 40 befinden, vorgesehen. Die RFID-Leseeinrichtung 44 kann wie in Figur 1 angedeutet neben dem ersten Bereich 40 angeordnet sein. Alternativ kann die RFID-Leseeinrichtung 44 beispielsweise unter der Ablagefläche 18 angeordnet sein. In diesem Fall ist die Ablagefläche 18 durch ein Bauteil gebildet, das für die elektrischen, magnetischen oder elektromagnetischen Wechselfelder, über die die RFID-Leseeinrichtung 44 mit RFID-Etiketten kommuniziert, transparent ist oder sie nicht wesentlich abschwächt.

Bei dem in Figur 1 dargestellten Beispiel weist das medizinische Instrument kein RFID-Etikett auf.

Ein zweiter Bereich 50 ist für die Bestückung eines Instrumententrägers 20 vorgesehen. In Figur 1 ist der Instrumententräger 20 in der vorgesehenen Position und Orientierung in dem zweiten Bereich 50 angeordnet. Die vorgesehene Position und Orientierung des Instrumententrägers 20 kann durch Anschläge mechanisch definiert sein.

Der zweite Bereich 50 und in diesem angeordnete Objekte werden von einer zweiten Kamera 52 optisch erfasst. Die zweite Kamera 52 erfasst dabei insbesondere den Instrumententräger 20 und auf dem Instrumententräger 20 abgelegte medizinische Instrumente 10. Optional kann die zweite Kamera 52 optisch lesbare Codes 12 an medizinischen Instrumenten und den optisch lesbaren Code 22 an dem Instrumententräger 20 erfassen.

Nahe dem zweiten Bereich 50 ist ferner eine RFID-Schreibeinrichtung 54 zum Schreiben von Daten in RFID-Etiketten, die sich in dem zweiten Bereich 50 befinden, vorgesehen. Die RFID-Schreibeinrichtung 54 kann gleichzeitig eine RFID-Leseeinrichtung zum Lesen von Daten aus RFID-Etiketten sein. Die RFID-Schreibeinrichtung 54 kann wie in Figur 1 angedeutet neben dem zweiten Bereich 50 angeordnet sein. Alternativ kann die RFID-Schreibeinrichtung 54 unter der vorgesehenen Position des Instrumententrägers 20 angeordnet sein. In diesem Fall ist die Ablagefläche, auf der der Instrumententräger 20 anzuordnen ist, durch ein Bauteil gebildet, das für die elektrischen, magnetischen oder elektromagnetischen Wechselfelder, über die die RFID-Schreibeinrichtung 54 mit RFID-Etiketten kommuniziert, transparent ist oder sie nicht wesentlich abschwächt.

Eine erste Lichtquelle 56 erzeugt Licht, das von dem photoelektrischen Bauteil 26 des RFID-Etiketts 24 an dem Instrumententräger 20 in elektrische Leistung gewandelt werden kann. Dazu ist das Emissionsspektrum der ersten Lichtquelle 56 insbesondere auf das Absorptionsspektrum des photoelektrischen Bauteils 26 abgestimmt, so dass ein möglichst großer Teil der der ersten Lichtquelle 56 zugeführten elektrischen Leistung durch das photoelektrische Bauteil 26 wieder in elektrische Leistung gewandelt wird.

Die erste Lichtquelle 56 erzeugt insbesondere nur einen schmalen Lichtstrahl oder Lichtkegel und beleuchtet vor allem den Bereich, in dem bei der vorgesehenen Anordnung des Instumententrägers 20 das photoelektrische Bauteil 26 angeordnet ist. Die erste Lichtquelle 56 umfasst insbesondere einen oder mehrere Laser oder Leuchtdioden.

Eine Projektionseinrichtung 58 projiziert den Umriss 28 des medizinischen Instruments 10 in der vorgesehenen Anordnung auf den Instrumententräger 20. Die Projektionseinrichtung 58 umfasst insbesondere einen Laser und eine Einrichtung zum steuerbaren Lenken des Laserstrahls, damit dieser den Umriss 28 beschreibt. Alternativ kann die Projektionseinrichtung 58 eine andere Lichtquelle und beispielsweise ein steuerbares Mikrospiegelarray, eine steuerbare Flüssigkristallmatrix oder ein Dia umfassen.

Ein dritter Bereich 60 ist für die Bestückung eines Sterilbehälters 30 mit einem oder mehreren Instrumententrägern 20 vorgesehen. In Figur 1 ist der Sterilbehälter 30 in der vorgesehenen Position und Orientierung in dem dritten Bereich 60 angeordnet.

Die vorgesehene Position und Orientierung des Sterilbehälters 30 kann durch Anschläge mechanisch definiert sein.

Der dritte Bereich 60 und in diesem angeordnete Objekte werden von einer dritten Kamera 62 optisch erfasst. Die dritte Kamera 62 erfasst dabei insbesondere den Sterilbehälter 30 den darin abgelegten Instrumententräger 20 (im Fall eines Stapels von Instrumententrägern: den obersten Instrumententräger 20) und auf dem Instrumententräger 20 abgelegte medizinische Instrumente 10. Optional kann die dritte Kamera 62 optisch lesbare Codes 12 an medizinischen Instrumenten und den optisch lesbaren Code 22 an dem Instrumententräger 20 erfassen.

Optional kann eine in Figur 1 nicht dargestellte weitere Einrichtung zum Erfassen des optisch lesbaren Codes 32 an dem Sterilbehälter 30 vorgesehen sein.

Nahe dem dritten Bereich 60 ist ferner eine RFID-Lese- und -Schreibeinrichtung 64 zum Lesen von Daten aus RFID-Etiketten und zum Schreiben von Daten in RFID-Etiketten, die sich in dem dritten Bereich 60 befinden, vorgesehen. Die RFID-Lese- und -Schreibeinrichtung 64 kann wie in Figur 1 angedeutet neben dem zweiten Bereich 50 angeordnet sein. Alternativ kann die RFID-Lese- und -Schreibeinrichtung 64 unter der vorgesehenen Position des Sterilbehälters 30 angeordnet sein. In diesem Fall ist die Ablagefläche, auf der der Sterilbehälter 30 anzuordnen ist, durch ein Bauteil gebildet, das für die elektrischen, magnetischen oder elektromagnetischen Wechselfelder, über die die RFID-Schreibeinrichtung 54 mit RFID-Etiketten kommuniziert, transparent ist oder sie nicht wesentlich abschwächt.

Eine zweite Lichtquelle 66 erzeugt Licht, das von dem photoelektrischen Bauteil 26 des RFID-Etiketts 24 an dem Instrumententräger 20 in elektrische Leistung gewandelt werden kann. Die zweite Lichtquelle 66 ähnelt in ihren Merkmalen, Eigenschaften und Funktionen insbesondere der ersten Lichtquelle 56 für das photoelektrische Bauteil 26 an dem Instrumententräger 20 in dem zweiten Bereich.

Eine dritte Lichtquelle 68 erzeugt Licht, das von dem photoelektrischen Bauteil 36 des RFID-Etiketts 34 an dem Sterilbehälter 30 in elektrische Leistung gewandelt werden kann. Die dritte Lichtquelle 68 ähnelt in ihren Merkmalen, Eigenschaften und Funktionen insbesondere der ersten Lichtquelle 56 und der zweiten Lichtquelle 66. Das Spektrum der dritten Lichtquelle 68 und der von der dritten Lichtquelle 68 beleuchtete Bereich sind dabei auf die spektralen Eigenschaften und die vorgesehene Anordnung des photoelektrischen Bauteils 36 an dem RFID-Etikett 34 an dem Sterilbehälter 30 abgestimmt.

Ein Computer 70 ist über in Figur 1 angedeutete elektrische oder optische Datenleitungen mit der ersten Kamera 42, der RFID-Leseeinrichtung 44, der zweiten Kamera 52, der RFID-Schreibeinrichtung 54, der Projektionseinrichtung 58, der dritten Kamera 62, der RFID-Lese- und -Schreibeinrichtung 64 verbunden. Optional kann der Computer 70 wie in Figur 1 angedeutet durch weitere elektrische oder optische Leitungen mit der ersten Lichtquelle 56, der zweiten Lichtquelle 66 und der dritten Lichtquelle 68 verbunden sein.

Der Computer 70 ist über in Figur 1 angedeutete elektrische oder optische Datenleitungen ferner mit einer Eingabeeinrichtung 72 (in Figur 1 als Tastatur dargestellt), einer Ausgabeeinrichtung 74 (in Figur 2 als Bildschirm oder Display dargestellt) und einer Datenbank 76 verbunden. Die Eingabeinrichtung 72 und die Ausgabeeinrichtung 74 bilden zusammen eine Benutzerschnittstelle.

In Figur 1 sind Punkt-zu-Punkt-Verbindungen angedeutet. Alternativ ist eine andere Topolgie möglich, beispielsweise eine Busstruktur, eine ringförmige oder eine sternförmige Topologie. Ferner können alternativ zu jedem einzelnen kabelgebundenen Kommunikationskanal jeweils eine Funkverbindung vorgesehen sein, beispielsweise entsprechend den Standards von WLAN, Bluetooth, ZigBee etc..

Abweichend von der Darstellung in Figur 1 können jeweils mehrere Einrichtungen in einem Gehäuse integriert sein. Beispielsweise kann die Datenbank 76, die Eingabeeinrichtung 72 und/oder die Ausgabeeinrichtung in den Computer 70 integriert sein.

Der Computer 70 ist vorgesehen und ausgebildet zum Auswerten eines Bildsignals der ersten Kamera 42, zum Identifizieren eines in dem durch das Bildsignal repräsentierten Bild abgebildeten medizinischen Instruments 10, zum Erzeugen entsprechender Identitätsdaten und zum Ermitteln einer Soll-Anordnung des identifizierten Instruments 10 auf dem Instrumententräger 20. Der Computer 70 ist ferner vorgesehen und ausgebildet, um die Projektionseinrichtung 58 so zu steuern, dass sie einen Umriss 28 des identifizierten Instruments 10 in der ermittelten Soll-Anordnung auf den Instrumententräger 20 projiziert oder die Soll-Anordnung in anderer Weise darstellt, beispielsweise auf der Ausgabeeinrichtung 74.

Der Computer 70 ist ferner vorgesehen und ausgebildet, um ein Bildsignal, das ein von der zweiten Kamera 52 optisch erfasstes Bild repräsentiert, zu empfangen, in dem Bild abgebildete medizinische Instrumente 10 zu identifizieren, entsprechende Identitätsdaten zu erzeugen, die Anordnung der identifizierten medizinischen Instrumente 10 relativ zu dem Instrumententräger 20 zu ermitteln, die Identitäten der identifizierten medizinischen Instrumente mit einer Soll-Bestückung zu vergleichen, die ermittelte Anordnung der identifizierten medizinischen Instrumente 10 mit einer Soll-Anordnung zu vergleichen und abhängig von diesen Vergleichen ein Meldesignal zu erzeugen.

Der Computer 70 ist ferner vorgesehen und ausgebildet, um ein Bildsignal, das ein von der zweiten Kamera 52 optisch erfasstes Bild repräsentiert, zu empfangen, in dem Bild abgebildete medizinische Instrumente 10 zu identifizieren, entsprechende Identitätsdaten zu erzeugen, die Anordnung der identifizierten medizinischen Instrumente 10 relativ zu dem Instrumententräger 20 zu ermitteln und als Soll-Anordnung zu speichern.

Der Computer 70 ist ferner vorgesehen und ausgebildet, um ein Bildsignal, das ein von der dritten Kamera 62 optisch erfasstes Bild repräsentiert, zu empfangen, in dem Bild abgebildete medizinische Instrumente 10 zu identifizieren, entsprechende Identitätsdaten zu erzeugen, die Anordnung der identifizierten medizinischen Instrumente 10 relativ zu dem Instrumententräger 20 zu ermitteln, die Identitäten der identifizierten medizinischen Instrumente mit einer Soll-Bestückung zu vergleichen, die ermittelte Anordnung der identifizierten medizinischen Instrumente 10 mit einer Soll-Anordnung zu vergleichen und abhängig von diesen Vergleichen ein Meldesignal zu erzeugen.

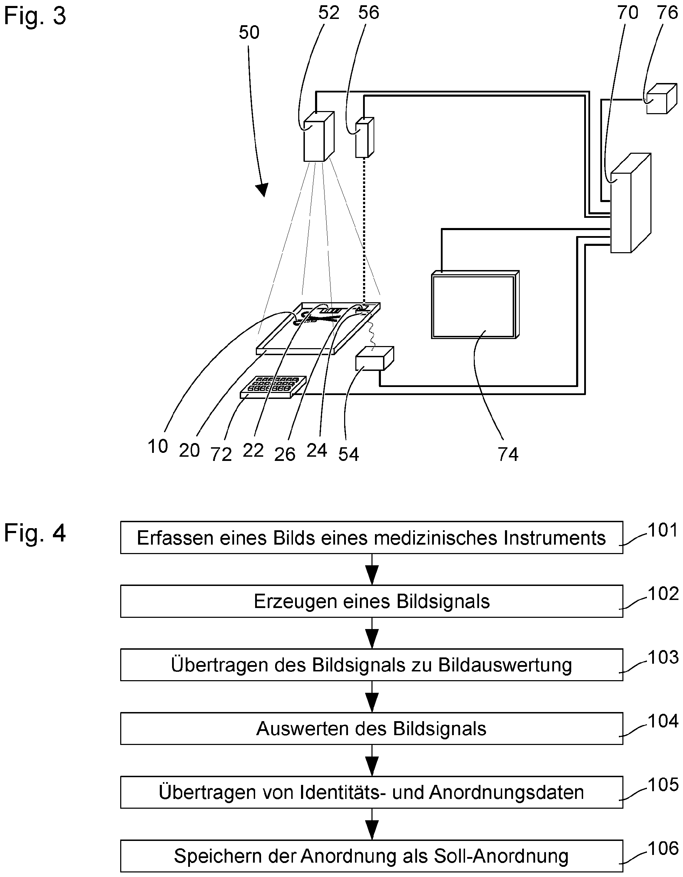

Dazu weist der Computer 70 insbesondere entsprechende Software auf, die insbesondere die anhand der Figuren 4 bis 6 beschriebenen Verfahrensschritte steuert.

Abweichend von der Darstellung in Figur 1 können anstelle dreier Bereiche 40, 50, 60 nur zwei Bereiche oder nur ein Bereich vorgesehen sein. Entsprechend können nur zwei Kameras oder nur eine Kamera und nur zwei oder nur eine RFID-Schreib- und Leseeinrichtungen vorgesehen sein. In diesem Fall sind die zwei Bereiche oder ist der eine Bereich groß genug, um nebeneinander ein medizinisches Instrument 10 und den Instrumententräger 20 oder nebeneinander den Instrumententräger 20 und den Sterilbehälter 30 oder nebeneinander das medizinische Instrument 10, den Instrumententräger 20 und den Sterilbehälter 30 aufzunehmen. Dabei kann das System nur zum optischen Erfassen des medizinischen Instruments 10 und des Instrumententrägers 20 oder nur zum optischen Erfassen des Instrumententrägers 20 und des Sterilbehälters 30 oder zum optischen Erfassen des medizinischen Instruments 10 und des Instrumententrägers 20 und des Sterilbehälters 30 vorgesehen und ausgebildet sein. Im selben Bereich können nacheinander ein medizinisches Instrument 10 und ein Instrumententräger 20 oder ein Instrumententräger 20 und ein Sterilbehälter 30 oder ein medizinisches Instrument 10, ein Instrumententräger 20 und ein Sterilbehälter 30 durch eine Kamera optisch erfasst werden. Im selben Bereich können durch eine einzige RFID-Schreib- und Leseeinrichtung nacheinander RFID-Etiketten eines medizinischen Instruments 10 und eines Instrumententrägers 20 oder RFID-Etiketten eines Instrumententrägers 20 und eines Sterilbehälter 30 oder RFID-Etiketten eines medizinischen Instruments 10, eines Instrumententrägers 20 und eines Sterilbehälter 30 gelesen und/oder beschrieben werden.

Abweichend von der Darstellung in Figur 1 können ferner eine oder mehrere Einrichtungen zum - insbesondere horizontalen - Verschieben der Ablagefläche 18, des Instrumententrägers 20 und/oder des Sterilbehälters 30 zwischen den Bereichen 40, 50, 60 vorgesehen sein. Abweichend von der Darstellung in Figur 1 können eine oder mehrere Einrichtungen zum - insbesondere horizontalen - Verschieben der ersten Kamera 42, der RFID-Leseeinrichtung 44, der Kamera 52, der RFID-Schreibeinrichtung 54, der ersten Lichtquelle 56, der ersten Projektionseinrichtung 58, der dritten Kamera 62, der RFID-Schreibe- und -Leseeinrichtung 64, der zweiten Lichtquelle 66 und/oder der dritten Lichtquelle 68 vorgesehen sein, um die starre Zuordnung der Bereiche 40, 50, 60 zu deren oben beschriebenen Funktionen aufzulösen.

Figur 2 zeigt eine schematische und vergrößerte Darstellung einer Dateneinrichtung, nämlich des RFID-Etiketts 24 oder des - insbesondere baugleichen - RFID-Etiketts 34 jeweils ohne den Instrumententräger oder den Sterilbehälter. Die Darstellung zeigt in einem angedeuteten quaderförmigen und transparent dargestellten Gehäuse eine Leistungsversorgungseinrichtung 82, einen Mikrocontroller 84, einen Datenspeicher 86 und eine Sende- und Empfangseinrichtung 88.

Die Leistungsversorgungseinrichtung 82 ist einerseits mit dem photoelektrischen Bauteil 26, 36 verbunden und bezieht von diesem elektrische Leistung. Die Leistungsversorgungseinrichtung 82 erzeugt insbesondere ein konstantes vorbestimmtes Spannungsniveau. Dazu kann die Leistungsversorgungseinrichtung 82 einen Energiespeicher enthalten, der Leistungsspitzen - beispielsweise aufgrund gepulster Lichtleistung - und Lastspitzen ausgleicht. Die Leistungsversorgungseinrichtung 82 stellt dem Mikrocontroller 84 und über diesen auch dem Datenspeicher 86 und der Sende- und Empfangseinrichtung 88 elektrische Leistung zur Verfügung. Dazu ist die Leistungversorgungseinrichtung 82 mit dem Mikrocontroller 84 verbunden. Abweichend von der Darstellung in Figur 2 kann die Leistungsversorgungseinrichtung 82 auch mit dem Datenspeicher 86 und/oder der Sende- und Empfangseinrichtung 88 unmittelbar verbunden sein, um diesen unmittelbar elektrische Leistung zur Verfügung zu stellen.

Der Mikrocontroller 84 ist durch Datenleitungen mit dem Datenspeicher 86 und der Sende- und Empfangseinrichtung 88 verbunden. Der Mikrocontroller 84 übermittelt zu speichernde Daten oder die Adressen von zu lesenden Daten an den Datenspeicher 86 und zu sendende Daten an die Sende- und Empfangseinrichtung 88. Der Mikrocontroller 84 empfängt aus dem Datenspecher 86 gelesene Daten und von der Sende- und Empfangseinrichtung 88 empfangene Daten.

Der Datenspeicher 86 ist insbesondere ein nicht-flüchtiger Datenspeicher, der gespeicherte Daten auch ohne Leistungsversorgung hält. Der Datenspeicher 86 ist zum Speichern von Identitätsdaten vorgesehen und ausgebildet. Der Datenspeicher 86 des mit dem Instrumententräger 20 dauerhaft verbundenen RFID-Etiketts 24 ist zum Speichern der Identitätsdaten der medizinischen Instrumente 10 auf dem Instrumententräger 20 und optional auch der Identität des Instrumententrägers 20 selbst vorgesehen. Der Datenspeicher 86 des mit dem Sterilbehälter 30 dauerhaft verbundenen RFID-Etiketts 34 ist zum Speichern der Identitätsdaten der medizinischen Instrumente 10 auf dem oder den Instrumententrägern 20 in dem Sterilbehälter 30 und optional auch der Identitätsdaten des oder der Instrumententräger 20 und/oder des Sterilbehälters 30 selbst vorgesehen. Beim Lesen von Identitätsdaten aus dem Datenspeicher 86 können alle gespeicherten Identitätsdaten oder einzeln adressierte Identitätsdaten ausgelesen werden.

Die Sende- und Empfangseinrichtung 88 ist in Figur 1 schematisch als planare Spule angedeutet. Neben einer solchen Antenne kann die Sende- und Empfangseinrichtung 88 Verstärker, Filter, Digital-Analog-Wandler und Analog-Digital-Wandler etc. umfassen. Diese können alternativ in den Mikrocontroller 84 integriert sein. Die Sende- und Empfangseinrichtung 88 entspricht einem RFID-Standard und sendet und empfängt Daten induktiv, kapazitiv oder in Form elektromagnetischer Wellen.

Solange das photoelektrische Bauteil 26, 36 ausreichend Leistung empfängt ist das RFID-Etikett 24, 34 nicht auf den Entnahme von Leistung aus dem magnetischen, elektrischen oder elektromagnetischen Wechselfeld angewiesen. Das RFID-Etikett 24, 34 kann vorgesehen und ausgebildet sein, um ohne Leistungsversorgung durch das photoelektrische Bauteil 26, 36 eine Mindestfunktionalität anzubieten. Beispielsweise umfasst die Mindestfunktionalität das Senden von Identitätsdaten, die die Identität des RFID-Etiketts 24, 34 und des Instrumententrägers 20 oder des Sterilbehälters 30 selbst umfassen, wenn ein Anforderungssignal ausreichender Stärke und ausreichenden Energiegehalts empfangen wurde.

Figur 3 zeigt eine schematische Darstellung eines Systems zur Unterstützung der Handhabung medizinischer Instrumente 10 insbesondere während einer medizinischen Maßnahme. Alternativ ist das in Figur 3 gezeigte System beispielsweise zur Unterstützung der Handhabung medizinischer Instrumente 10 nach der Reinigung, unmittelbar vor oder nach der Sterilisation und vor der Verwendung, insbesondere während des Bestückens verwendbar. Das System ähnelt dem anhand der Figur 1 dargestellten System. Es ist jedoch nur der zweite Bereich 50 mit der zweiten Kamera 52 und der ersten Lichtquelle 56 zur Bereitstellung von Lichtleistung für das photoelektrische Bauelement 26 an einem mit einem Instrumententräger 20 dauerhaft mechanisch verbundenen RFID-Etikett 24 vorgesehen. Der erste Bereich 40 mit der ersten Kamera 42 und der RFID-Leseeinrichtung 44 und der dritte Bereich 60 mit der dritten Kamera 62, der dritten RFID-Schreib- und Leseeinrichtung 64, der zweiten Lichtquelle 66 und der dritten Lichtquelle 68 sind nicht vorgesehen.

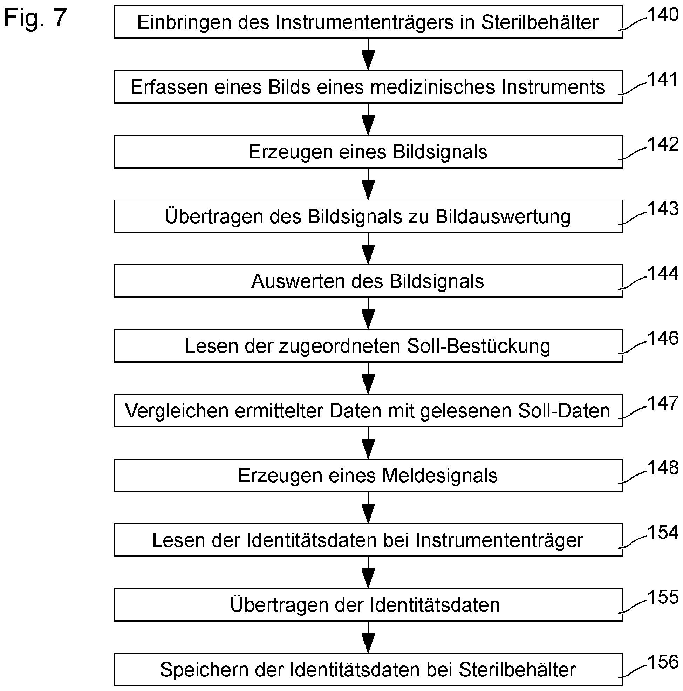

Das System in Figur 3 gezeigte System ist insbesondere zur Unterstützung der Handhabung medizinischer Instrumente 10 während einer medizinischen Maßnahme vorgesehen und ausgebildet. In dem Bereich 50 wird dazu ein Instrumententräger 20 angeordnet, auf dem ein oder mehrere medizinische Instrumente 10 zur Verwendung bei der medizinischen Maßnahme bereitgehalten werden. Die Kamera 52 ist zur optischen Erfassung der auf dem Instrumententräger 20 angeordneten medizinischen Instrumente 10 vorgesehen und angeordnet. Die RFID-Leseeinrichtung 54 ist zum Lesen von Identitätsdaten aus dem Speicher des mit dem Instrumententräger 20 dauerhaft verbundenen RFID-Etiketts 24 vorgesehen und ausgebildet. Der Computer 70 ist vorgesehen und ausgebildet zum Auswerten eines Bildsignals der Kamera 52, zum Identifizieren von medizinischen Instrumenten 10, die in dem von der Kamera 52 erfassten Bild abgebildet sind, und Erzeugen entsprechender Identitätsdaten, zum Vergleichen der erzeugten Identitätsdaten mit aus dem Speichr des RFID-Etiketts 24 ausgelesenen Identitätsdaten und zum Ändern eines Attributs in Identitätsdaten zu einem medizinischen Instrument abhängig von dem Vergleich. Dazu weist der Computer 70 insbesondere entsprechende Software auf, die insbesondere die anhand der Figur 7 beschriebenen Verfahrensschritte steuert.

Figur 4 zeigt ein schematisches Flussdiagramm eines Verfahrens zur Unterstützung der Handhabung medizinischer Instrumente. Das Verfahren ist mit dem anhand der Figur 1 dargestellten System oder mit dem anhand der Figur 3 dargestellten System und insbesondere auch mit dem anhand der Figur 2 dargestellten RFID-Etikett 24, 34 ausführbar. Das Verfahren ist jedoch auch mit einem System und mit einem Instrumententräger ausführbar, die von den Darstellungen anhand der Figuren 1, 2, 3 abweichende Merkmale, Eigenschaften und Funktionen aufweisen. Deshalb ist die nachfolgende Verwendung von Bezugszeichen aus den Figuren 1, 2, 3 lediglich beispielhaft illustrierend.

Bei einem ersten Schritt 101 wird ein Bild eines oder mehrerer medizinischer Instrumente 10 auf einem Instrumententräger 20 durch eine Kamera 52 optisch erfasst. Die Kamera 52 erfasst das Bild insbesondere im infraroten Spektralbereich und/oder im für das gesunde menschliche Auge sichtbaren Spektralbereich und/oder im ultravioletten Spektralbereich Die Kamera 42, 52 kann das Bild monochromatisch oder gleichzeitig in mehreren Farbkanälen erfassen. Die Kamera 42, 52 kann monokular oder eine Stereokamera sein.

Bei einem nachfolgenden Schritt 102 wird ein analoges oder digitales Bildsignal, das das erfasste Bild repräsentiert, erzeugt. Dies geschieht beispielsweise in einem oder mehreren Bildsensoren der Kamera 52.

Bei einem nachfolgenden Schritt 103 wird das Bildsignal zu einer Bildsignalauswertungseinrichtung 70 übertragen. Die Übertragung erfolgt insbesondere durch elektrische oder optische Kabel oder über WLAN, Bluetooth, ZigBee oder eine andere Funkverbindung. Insoweit die Bildauswertungseinrichtung 70 in die Kamera 52 integriert ist, kann die Übertragung entfallen.

Bei einem nachfolgenden Schritt 104 wird das Bildsignal durch die Bildsignalauswertungseinrichtung 70 ausgewertet, um die Identität des Instrumententrägers 20, die Identitäten und die Anordnung der in dem erfassten Bild abgebildeten medizinischen Instrumente 10 relativ zu dem Instrumententräger 20 zu ermitteln. Die Anordnung der medizinischen Instrumente umfasst insbesondere auch deren Orientierung.

Zur Ermittlung der Identität des Instrumententrägers 20 kann gegebenenfalls ein optisch lesbarer Code an dem Instrumententräger 20 gelesen werden. Alternativ oder zusätzlich kann die Identität des Instrumententrägers 20 aus einem mit dem Instrumententräger 20 dauerhaft mechanisch verbundenen RFID-Etikett 24 ausgelesen werden. Alternativ oder zusätzlich kann die Identität des Instrumententrägers 20 an einer Eingabeeinrichtung 72 manuell eingegeben werden.

Die Identität jedes abgebildeten medizinischen Instruments 10 wird beispielsweise anhand eines optisch lesbaren Codes 12 an dem medizinischen Instrument 10 und/oder anhand optisch erkennbarer Merkmale wie der Größe, der Gestalt und/oder Farben ermittelt. Dazu wird insbesondere eine Datenbank, in der entsprechende Merkmale abgelegt und einzelnen Typen medizinischer Instrumente oder einzelnen individuellen medizinischen Instrumenten zugeordnet sind, verwendet. Falls ein medizinisches Instrument 10 ein RFID-Etikett aufweist, kann alternativ oder zusätzlich das RFID-Etikett durch eine RFID-Leseeinrichtung 44 ausgelesen werden, um die Identität des medizinischen Instruments 10 zu ermitteln.

Dabei erzeugt die Bildsignalauswertungseinrichtung 70 Identitätsdaten, die die Identitäten der abgebildeten medizinischen Instrumente 10 repräsentieren, und Anordnungsdaten, die die Anordnung der abgebildeten medizinischen Instrumente 10 relativ zu dem Instrumententräger 20 repräsentieren.

Die Bildsignalauswertungseinrichtung 70 ist insbesondere ein Computer mit einer Software zur Bildauswertung.

Bei einem nachfolgenden Schritt 105 werden die Identitätsdaten und die Anordnungsdaten zu einem Datenspeicher 76, 86 übertragen. Der Datenspeicher kann in die Bildauswertungseinrichtung 70 integriert sein, beispielsweise als Massenspeicher. Alternativ kann der Datenspeicher eine von der Bildauswertungseinrichtung 70 separate Vorrichtung 76 oder in einer von der Bildauswertungseinrichtung 70 separaten Vorrichtung vorgesehen sein. Beispielsweise ist der Datenspeicher 76 Teil einer Datenbank in einem Server eines Krankenhauses oder einer anderen medizinischen Einrichtung. In diesem Fall erfolgt die Übertragung insbesondere über elektrische oder optische Kabel oder über WLAN, Bluetooth, ZigBee oder eine andere Funkverbindung.

Alternativ werden die Identitätsdaten und die Anordnungsdaten zu einem Datenspeicher 86 in einem mit dem Instrumententräger 20 dauerhaft mechanisch verbundenen RFID-Etikett 20 übertragen. In diesem Fall erfolgt die Übertragung insbesondere entsprechend einem RFID-Standard. Ferner kann in diesem Fall die Ermittlung der Identität des Instrumententrägers 20 im Schritt 104 entfallen.

Bei einem nachfolgenden Schritt 106 werden die Identitätsdaten und die Anordnungsdaten in dem Datenspeicher 76, 86 gespeichert.

Die gespeicherten Identitätsdaten und Anordnungsdaten repräsentieren die Soll-Bestückung des Instrumententrägers 20 und die Soll-Anordnung der medizinischen Instrumente 10 auf dem Instrumententräger 20. Bei jeder nachfolgenden Bestückung des Instrumententrägers 20 können die gespeicherten Identitätsdaten und Anordnungsdaten zur Steuerung der Bestückung des Instrumententräger 20 durch eine Person oder eine Vorrichtung verwendet werden. Bei jeder nachfolgenden Bestückung des Instrumententrägers 20 kann die tatsächlich erzeugte Bestückung und Anordnung der medizinischen Instrumente 10 auf dem Instrumententräger 20 mit den gespeicherten Identitätsdaten und den gespeicherten Anordnungsdaten verglichen werden, um zu prüfen, ob der Instrumententräger 20 korrekt bestückt ist.

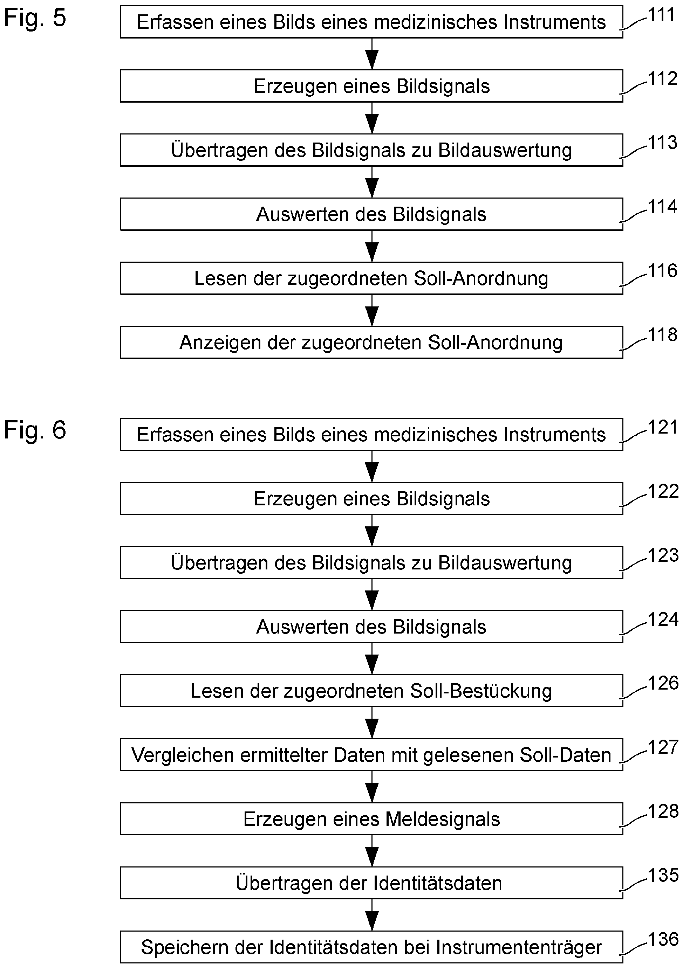

Figur 5 zeigt ein schematisches Flussdiagramm eines weiteren Verfahrens zur Unterstützung der Handhabung medizinischer Instrumente. Das Verfahren ist mit dem anhand der Figur 1 dargestellten System und insbesondere auch mit dem anhand der Figur 2 dargestellten RFID-Etikett 24, 34 ausführbar. Das Verfahren ist jedoch auch mit einem System und mit einem Instrumententräger ausführbar, die von den Darstellungen anhand der Figuren 1, 2 abweichende Merkmale, Eigenschaften und Funktionen aufweisen. Deshalb ist die nachfolgende Verwendung von Bezugszeichen aus den Figuren 1, 2 lediglich beispielhaft illustrierend.

Mehrere Schritte des in Figur 5 gezeigten Verfahrens ähneln Schritten des anhand der Figur 4 dargestellten Verfahrens und können die anhand der Figur 4 beschriebenen Merkmale und Eigenschaften aufweisen und auf die anhand der Figur 4 beschriebene Weise ausgeführt werden. Entsprechendes gilt für die erforderlichen oder benutzten Vorrichtungen und deren Merkmale, Eigenschaften und Funktionen, die den anhand der Figur 4 dargestellten ähneln oder entsprechen können.

Bei einem ersten Schritt 111 wird ein Bild eines medizinischen Instruments 10 durch eine Kamera 42 optisch erfasst. Das medizinische Instrument 10 liegt dabei insbesondere auf einer Ablagefläche 18. Alternativ kann das medizinische Instrument 10 manuell in das Sichtfeld der Kamera 42 gehalten werden. Alternativ kann das medizinische Instrument 10 auf einem Instrumententräger 20 liegen während die Kamera 42 das Bild erfasst.

Bei einem nachfolgenden Schritt 112 wird ein analoges oder digitales Bildsignal, das das erfasste Bild repräsentiert, erzeugt.

Bei einem nachfolgenden Schritt 113 wird das Bildsignal zu einer Bildsignalauswertungseinrichtung 70 übertragen.

Bei einem nachfolgenden Schritt 114 wird das Bildsignal durch die Bildsignalauswertungseinrichtung 70 ausgewertet, um die Identität des in dem erfassten Bild abgebildeten medizinischen Instruments 10 zu ermitteln. Dabei erzeugt die Bildsignalauswertungseinrichtung Identitätsdaten, die die Identität des abgebildeten medizinischen Instruments 10 repräsentieren. Falls ein medizinisches Instrument 10 ein RFID-Etikett aufweist, kann alternativ oder zusätzlich dieses durch eine RFID-Leseeinrichtung 44 ausgelesen werden, um die Identität des medizinischen Instruments 10 zu ermitteln.

Bei einem nachfolgenden Schritt 116 wird die dem identifizierten medizinischen Instrument 10 zugeordnete Soll-Anordnung auf einem Instrumententräger 20 ermittelt. Dabei werden Anordnungsdaten, die die Soll-Anordnung des identifizierten medizinischen Instruments repräsentieren, aus einem Datenspeicher 76, 86 gelesen. Für den Datenspeicher gilt das zu Figur 4 Ausgeführte. Insbesondere handelt es sich um denselben Datenspeicher 76, 86, in dem die Soll-Anordnung zuvor mit den anhand der Figur 4 dargestellten Schritten gespeichert wurde.

Bei einem nachfolgenden Schritt 118 wird die durch die Anordnungsdaten repräsentierte ermittelte Soll-Anordnung des medizinischen Instruments 10 angezeigt. Insbesondere projiziert eine Projektionseinrichtung 58 die Soll-Anordnung auf den Instrumententräger 20. Alternativ kann die Soll-Anordnung beispielsweise durch einen Bildschirm oder eine andere Ausgabeeinrichtung 74 angezeigt werden.

Die anhand der Figur 5 dargestellten Verfahrensschritte können die Bestückung eines Instrumententrägers 20 vereinfachen und dadurch und dabei auch die Fehlerquote reduzieren.

Einige der anhand der Figur 5 dargestellten Schritte sind optional. Beispielsweise können die Schritte 111, 112, 113, 114 entfallen, wenn das medizinische Instrument 10 ein RFID-Etikett aufweist. In diesem Fall wird stattdessen das RFID-Etikett des medizinischen Instruments 10 ausgelesen, um seine Identität zu ermitteln. Alternativ werden sowohl die Schritte 111, 112, 113, 114 ausgeführt als auch das RFID-Etikett des medizinischen Instruments 10 ausgelesen, um durch diese Redundanz eine erhöhte Sicherheit in der Bestimmung der Identität des medizinischen Instruments 10 zu erhalten.

Figur 6 zeigt ein schematisches Flussdiagramm eines weiteren Verfahrens zur Unterstützung der Handhabung medizinischer Instrumente. Das Verfahren ist mit dem anhand der Figur 1 dargestellten System, mit dem anhand der Figur 3 dargestellten System und insbesondere auch mit dem anhand der Figur 2 dargestellten RFID-Etikett 24, 34 ausführbar. Das Verfahren ist jedoch auch mit einem System und mit einem Instrumententräger ausführbar, die von den Darstellungen anhand der Figuren 1, 2, 3 abweichende Merkmale, Eigenschaften und Funktionen aufweisen. Deshalb ist die nachfolgende Verwendung von Bezugszeichen aus den Figuren 1, 2, 3 lediglich beispielhaft illustrierend.

Mehrere Schritte des in Figur 6 gezeigten Verfahrens ähneln Schritten der anhand der Figuren 4, 5 dargestellten Verfahren und können die anhand der Figuren 4, 5 beschriebenen Merkmale und Eigenschaften aufweisen und auf die anhand der Figuren 4, 5 beschriebene Weise ausgeführt werden. Entsprechendes gilt für die erforderlichen oder benutzten Vorrichtungen und deren Merkmale, Eigenschaften und Funktionen, die den anhand der Figuren 4, 5 dargestellten ähneln oder entsprechen können.

Bei einem ersten Schritt 121 wird ein Bild eines oder mehrerer medizinischer Instrumente 10 auf einem Instrumententräger durch eine Kamera 52 optisch erfasst.

Bei einem nachfolgenden Schritt 122 wird ein Bildsignal, das das erfasste Bild repräsentiert, erzeugt.

Bei einem nachfolgenden Schritt 123 wird das Bildsignal zu einer Bildsignalauswertungseinrichtung 70 übertragen.

Bei einem nachfolgenden Schritt 124 wird das Bildsignal durch die Bildsignalauswertungseinrichtung 70 ausgewertet, um die Identität des Instrumententrägers 20, die Identitäten und die Anordnung der in dem erfassten Bild abgebildeten medizinischen Instrumente 10 relativ zu dem Instrumententräger 20 zu ermitteln. Die Anordnung der medizinischen Instrumente 10 umfasst insbesondere auch deren Orientierung.

Die Ermittlung der Identität des Instrumententrägers 20 und die Ermittlung der Identitäten und Anordnung der medizinischen Instrumente 10 erfolgen insbesondere wie anhand der Figur 4 im Kontext des Schritts 104 dargestellt.

Bei der Ermittlung der Identitäten und Anordnung der medizinischen Instrumente 10 werden Bestückungsdaten erzeugt. Die Bestückungsdaten umfassen Identitätsdaten, die die Identitäten der abgebildeten medizinischen Instrumente 10 repräsentieren, und optional auch Anordnungsdaten, die die Anordnung der abgebildeten medizinischen Instrumente 10 relativ zu dem Instrumententräger 20 repräsentieren.

Bei einem nachfolgenden Schritt 126 werden - insbesondere durch die Bildauswertungseinrichtung 70 - eine dem identifizierten Instrumententräger 20 zugeordnete Soll-Bestückungsdaten aus einem Datenspeicher 76, 86 gelesen. Die Soll-Bestückungsdaten repräsentieren die Soll-Bestückung des identifizierten Instrumententrägers 20, umfassen also insbesondere die Identitätsdaten aller medizinischer Instrumente, die bei der Soll-Bestückung auf dem Instrumententräger angeordnet sind. Die Soll-Bestückungsdaten umfassen optional auch Soll-Anordnungsdaten, die die Soll-Anordnung der medizinischen Instrumente auf dem Instrumententräger 20 repräsentieren. Für den Datenspeicher 76 ,86 gilt das zu Figur 4 Ausgeführte. Insbesondere handelt es sich um denselben Datenspeicher 76, 86.

Bei einem nachfolgenden Schritt 127 werden - insbesondere durch die Bildauswertungseinrichtung 70 - die ermittelten Identitätsdaten mit der gelesenen Soll-Bestückung und die ermittelte Anordnung mit der gelesenen Soll-Anordnung verglichen.

Bei einem nachfolgenden Schritt 128 wird abhängig vom Vergleich in Schritt 127 ein Meldesignal erzeugt, das anzeigt, ob die ermittelte Bestückung der gelesenen Soll-Bestückung des Instrumententrägers 20 und die ermittelte Anordnung des oder der medizinischen Instrumente 10 auf dem Instrumententräger 20 der gelesenen Soll-Anordnung entspricht. Falls die ermittelte Bestückung nicht der gelesenen Soll-Bestückung oder die ermittelte Anordnung des oder der medizinischen Instrumente 10 auf dem Instrumententräger 20 nicht der gelesenen Soll-Anordnung entspricht, kann das Meldesignal ferner Information über die Abweichung und/oder Anweisungen für eine Korrektur umfassen. Im Fall einer korrekten Bestückung und Anordnung kann das Meldesignal den Transport des Instrumententrägers 20 zu einer nächsten Behandlungsstation auslösen oder steuern.

Das Meldesignal kann von der Ausgabeeinrichtung 74 erzeugt werden und unmittelbar von einer Person akustisch oder optisch oder taktil wahrnehmbar sein. Alternativ kann das Meldesignal ein analoges oder digitales Signal sein, das an eine Ausgabeeinrichtung 74 oder an eine andere Vorrichtung übertragen wird. Das Meldesignal kann insbesondere an einen Industrieroboter oder mehrachsigen Bewegungsautomaten oder eine Steuerung für diesen übertragen werden. Im Fall einer fehlerhaften Bestückung und Anordnung kann das Meldesignal eine Korrektur der Bestückung oder der Anordnung durch den Industrieroboter auslösen oder steuern.

Bei einem nachfolgenden Schritt 135 werden die Identitätsdaten und optional auch die Anordnungsdaten der auf dem Instrumententräger 20 angeordneten medizinischen Instrumente 10 zu einem mit dem Instrumententräger 20 dauerhaft mechanisch verbundenen RFID-Etikett 24 übertragen. Dies erfolgt insbesondere durch eine RFID-Schreibeinrichtung 54 und entsprechend einem RFID-Standard.

Bei einem nachfolgenden Schritt 136 werden die übertragenen Identitätsdaten und optional auch die Anordnungsdaten der auf dem Instrumententräger 20 angeordneten medizinischen Instrumente 10 in einem Datenspeicher 86 des mit dem Instrumententräger 20 dauerhaft mechanisch verbundenen RFID-Etikett 24 gespeichert.

Mittels der anhand der Figur 6 dargestellten Verfahrensschritte kann die Bestückung eines Instrumententrägers 20 einschließlich der Anordnung der medizinischen Instrumente 10 darauf geprüft werden, beispielsweise zur Qualitätssicherung. Die anhand der Figur 6 dargestellten Verfahrensschritte werden dazu insbesondere nach den anhand der Figur 5 dargestellten Verfahrensschritten ausgeführt. Alternativ können die anhand der Figur 6 dargestellten Verfahrensschritte nach einer maschinellen Bestückung des Instrumententrägers 20 ausgeführt werden.

Einige der anhand der Figur 6 dargestellten Schritte sind optional. Beispielsweise können die Schritte 121, 122, 123, 124, 126, 127, 128 entfallen, wenn die Bestückung des Instrumententrägers 20 mit hinreichender Sicherheit bekannt und die Wahrscheinlichkeit einer fehlerhaften Bestückung hinreichend gering ist. Ferner können beispielsweise die Schritte 135, 136 entfallen und stattdessen Identitätsdaten in einem anderen Datenspeicher 76 abgelegt und dort dem Instrumententräger 20 zugeordnet werden.

Figur 7 zeigt ein schematisches Flussdiagramm eines weiteren Verfahrens zur Unterstützung der Handhabung medizinischer Instrumente. Das Verfahren ist mit dem anhand der Figur 1 dargestellten System, mit dem anhand der Figur 3 dargestellten System und insbesondere auch mit dem anhand der Figur 2 dargestellten RFID-Etikett 24, 34 ausführbar. Das Verfahren ist jedoch auch mit einem System und mit einem Instrumententräger ausführbar, die von den Darstellungen anhand der Figuren 1, 2, 3 abweichende Merkmale, Eigenschaften und Funktionen aufweisen. Deshalb ist die nachfolgende Verwendung von Bezugszeichen aus den Figuren 1, 2, 3 lediglich beispielhaft illustrierend.

Mehrere Schritte des in Figur 7 gezeigten Verfahrens ähneln Schritten der anhand der Figuren 4, 5 und vor allem 6 dargestellten Verfahren und können die anhand der Figuren 4, 5, 6 beschriebenen Merkmale und Eigenschaften aufweisen und auf die anhand der Figuren 4, 5, 6 beschriebene Weise ausgeführt werden. Entsprechendes gilt für die erforderlichen oder benutzten Vorrichtungen und deren Merkmale, Eigenschaften und Funktionen, die den anhand der Figuren 4, 5, 6 dargestellten ähneln oder entsprechen können.

Bei einem ersten Schritt 140 wird ein Instrumententräger 20 in einen Sterilbehälter 30 eingebracht. Vor dem ersten Schritt 140 kann der Instrumententräger 20 mit den anhand der Figur 5 dargestellten Schritten bestückt worden sein. Ferner kann die Bestückung des Instrumententrägers 20 vor dem ersten Schritt mit den anhand der Figur 6 dargestellten Schritten geprüft worden sein. Wenn der Sterilbehälter 30 zur Aufnahme mehrerer Instrumententräger 20 in einem Stapel vorgesehen ist, sind die nachfolgenden in Figur 7 gezeigten Verfahrensschritte insbesondere auf den zuletzt eingebrachten obersten Instrumententräger 20 bezogen.

Die nachfolgenden Schritte 141, 142, 143, 144, 146, 147, 148 entsprechen insbesondere weitgehend den anhand der Figur 6 dargestellten Schritten 121, 122, 123, 124, 126, 127, 128. Die Schritte 141, 142, 143, 144, 146, 147, 148 unterscheiden sich von den anhand der Figur 6 dargestellten Schritten 121, 122, 123, 124, 126, 127, 128 insbesondere nur dadurch, dass der Instrumententräger 20 in dem Sterilbehälter angeordnet ist. Die Schritte 141, 142, 143, 144, 146, 147, 148 dienen der Prüfung der korrekten Bestückung des Instrumententrägers 20 und der korrekten Anordnung der medizinischen Instrumente 10 in dem Instrumententräger 20.

Bei einem nachfolgenden Schritt 154 werden die Identitätsdaten und optional auch die Anordnungsdaten der auf dem Instrumententräger 20 angeordneten medizinischen Instrumente 10 aus dem mit dem Instrumententräger 20 dauerhaft mechanisch verbundenen RFID-Etikett 24 gelesen. Die Übertragung erfolgt insbesondere durch eine RFID-Schreib- und -Leseeinrichtung 54 und entsprechend einem RFID-Standard.

Bei einem nachfolgenden Schritt 155 werden die bei dem vorangehenden Schritt gelesenen Identitätsdaten und optional auch die Anordnungsdaten der auf dem Instrumententräger 20 angeordneten medizinischen Instrumente 10 zu einem mit dem Sterilbehälter 30 dauerhaft mechanisch verbundenen RFID-Etikett 34 übertragen. Dabei werden optional auch Identitätsdaten, die die Identität des Instrumententrägers 20 repräsentieren, zu einem mit dem Sterilbehälter 30 dauerhaft mechanisch verbundenen RFID-Etikett 34 übertragen. Die Übertragung erfolgt insbesondere durch die RFID-Schreib- und -Leseeinrichtung 54 und entsprechend einem RFID-Standard.

Bei einem nachfolgenden Schritt 156 werden die übertragenen Identitätsdaten und optional auch die Anordnungsdaten der auf dem Instrumententräger 20 angeordneten medizinischen Instrumente 10 in einem Datenspeicher 86 des mit dem Sterilbehälter 30 dauerhaft mechanisch verbundenen RFID-Etikett 34 gespeichert.

Mittels der anhand der Figur 7 dargestellten Verfahrensschritte kann die Bestückung eines Instrumententrägers 20 einschließlich der Anordnung der medizinischen Instrumente 10 darauf geprüft werden, beispielsweise zur Qualitätssicherung. Die anhand der Figur 7 dargestellten Verfahrensschritte werden dazu insbesondere nach den anhand der Figuren 5 und 6 dargestellten Verfahrensschritten ausgeführt. Alternativ können die anhand der Figur 7 dargestellten Verfahrensschritte nach einer maschinellen Bestückung des Instrumententrägers 20 oder des Sterilbehälters 30 ausgeführt werden.

Einige der anhand der Figur 7 dargestellten Schritte sind optional. Beispielsweise können die Schritte 141, 142, 143, 144, 146, 147, 148 entfallen, wenn die Bestückung des Instrumententrägers 20 mit hinreichender Sicherheit bekannt und die Wahrscheinlichkeit einer fehlerhaften Bestückung hinreichend gering ist.

Ferner kann der Schritt 154 entfallen, wenn die Identitätsdaten und optional auch die Anordnungsdaten in einem anderen Speicher 76 gespeichert sind. In diesem Fall können stattdessen die Identitätsdaten und optional auch die Anordnungsdaten aus dem anderen Speicher, beispielsweise einer zentralen Datenbank 76 gelesen werden.

Ferner können die Schritte 155, 156 entfallen. Stattdessen können die Identitätsdaten und optional auch die Anordnungsdaten der auf dem Instrumententräger 20 angeordneten medizinischen Instrumente 10 zusammen mit Identitätsdaten, die die Identität des Instrumententrägers 20 repräsentieren, in einem anderen Speicher, beispielsweise einer zentralen Datenbank 76 gespeichert werden.

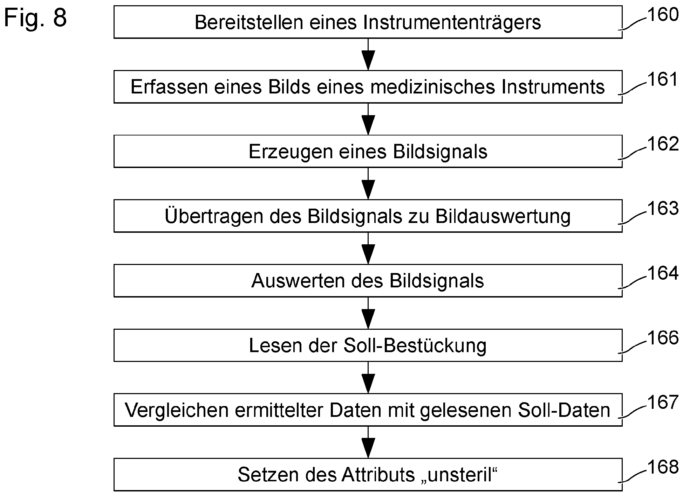

Figur 8 zeigt ein schematisches Flussdiagramm eines weiteren Verfahrens zur Unterstützung der Handhabung medizinischer Instrumente. Das Verfahren ist mit dem anhand der Figur 1 dargestellten System, mit dem anhand der Figur 3 dargestellten System und insbesondere auch mit dem anhand der Figur 2 dargestellten RFID-Etikett 24, 34 ausführbar. Das Verfahren ist jedoch auch mit einem System und mit einem Instrumententräger ausführbar, die von den Darstellungen anhand der Figuren 1, 2, 3 abweichende Merkmale, Eigenschaften und Funktionen aufweisen. Deshalb ist die nachfolgende Verwendung von Bezugszeichen aus den Figuren 1, 2, 3 lediglich beispielhaft illustrierend.

Mehrere Schritte des in Figur 8 gezeigten Verfahrens ähneln Schritten der anhand der Figuren 4, 5, 6, 7 dargestellten Verfahren und können die anhand der Figuren 4, 5, 6, 7 beschriebenen Merkmale und Eigenschaften aufweisen und auf die anhand der Figuren 4, 5, 6, 7 beschriebene Weise ausgeführt werden. Entsprechendes gilt für die erforderlichen oder benutzten Vorrichtungen und deren Merkmale, Eigenschaften und Funktionen, die den anhand der Figuren 4, 5, 6, 7 dargestellten ähneln oder entsprechen können.

Bei einem ersten Schritt 160 wird ein Instrumententräger 20 für die Verwendung bei einer medizinischen Maßnahme bereitgestellt. Die Bestückung des Instrumententrägers 20, also die Identitäten der darauf angeordneten medizinischen Instrumente 10 und optional auch deren Anordnung und Orientierung sind an die medizinische Maßnahme angepasst. Für die medizinische Maßnahme wird also ein Instrumententräger 20 mit geeigneter Bestückung ausgewählt.

Der bereitgestellte Instrumententräger 20 und die darauf angeordneten medizinischen Instrumente 10 sind bis zur Bereitstellung insbesondere steril. Dazu wurden sie insbesondere autoklaviert. Während der medizinischen Maßnahme werden medizinische Instrumente 10 von dem Instrumententräger 20 entnommen und verwendet. Sie sind dann nicht mehr steril.

Während der medizinischen Maßnahme werden die Schritte 161, 162, 163, 164, die weitgehend den anhand der Figur 6 dargestellten Schritten 121, 122, 123, 124 und den anhand der Figur 7 dargestellten Schritten 141, 142, 143, 144 entsprechen, ausgeführt. Sie werden insbesondere von und mit dem anhand der Figur 3 dargestellten System ausgeführt. Dabei ist bei dem Schritt 164 das Ermitteln der Anordnung der in dem erfassten Bild abgebildeten medizinischen Instrumente 10 optional.

Bei einem nachfolgenden Schritt 166 werden dem Instrumententräger 20 zugeordnete Soll-Bestückungsdaten, das heißt die Identitätsdaten der bei der Soll-Bestückung auf dem Instrumententräger 20 angeordneten medizinischen Instrumente aus einem Datenspeicher 76, 86 gelesen. Die Soll-Bestückungsdaten umfassen optional auch Soll-Anordnungsdaten, die die Soll-Anordnung der medizinischen Instrumente auf dem Instrumententräger 20 repräsentieren. Für den Datenspeicher 76, 86 gilt das zu Figur 4 Ausgeführte. Insbesondere handelt es sich um denselben Datenspeicher 76, 86.

Bei einem nachfolgenden Schritt 167 werden die ermittelten Bestückungsdaten mit den aus dem Speicher 76, 86 gelesenen Soll-Bestückungsdaten verglichen, beispielsweise durch die Bildauswertungseinrichtung 70.

Bei einem nachfolgenden Schritt 168 wird zu einem medizinischen Instrument 10, das einerseits Teil der Soll-Bestückung des Instrumententrägers 20 ist, aber andererseits nicht Teil der ermittelten Bestückung ist, das Attribut "unsteril" gesetzt. Dieses Attribut wird insbesondere in dem Speicher 86 des mit dem Instrumententräger 20 dauerhaft verbundenen RFID-Etiketts 24 und/oder in einer zentralen Datenbank 76 oder in einem andern Speicher gespeichert. Das Attribut "unsteril" kennzeichnet, dass das medizinische Instrument vor jeder weiteren Verwendung zu sterilisieren ist.

Bei dem Schritt 168 kann gleichzeitig ein Zähler, der die Anzahl der Verwendungen des medizinischen Instruments zählt, inkrementiert werden. Ein Vergleich des Zählers mit einer vorbestimmten Obergrenze von Verwendungen ermöglicht das rechtzeitige Ausscheiden des medizinischen Instruments aus dem Materialbestand eines Krankenhauses, einer Arztpraxis oder einer anderen medizinischen Einrichtung.

Während der medizinischen Maßnahme werden die Schritte 161, 162, 163, 164, 167, 168 insbesondere wiederholt ausgeführt, optional auch der Schritt 166.

### Bezugszeichen

- 10: medizinisches Instrument
- 12: optisch lesbarer Code an dem medizinischen Instrument 10
- 18: Ablagefläche für das medizinische Instrument 10
- 20: autoklavierbarer Instrumententräger (insbesondere Sieb, Tablett etc.)
- 22: optisch lesbarer Code an dem autoklavierbaren Instrumententräger 20
- 24: RFID-Etikett an dem autoklavierbaren Instrumententräger 20
- 26: photoelektrisches Bauelement an dem RFID-Etikett 24 an dem autoklavierbaren Instrumententräger 20 zum Empfang optischer Leistung und zum Bereitstellen elektrischer Leistung für das RFID-Etikett 24
- 28: auf den autoklavierbaren Instrumententräger 20 projizierter Umriss des medizinischen Instruments 10 in einer Soll-Position
- 30: autoklavierbarer Sterilbehälter
- 32: optisch lesbarer Code an dem autoklavierbaren Sterilbehälter 30
- 34: RFID- Etikett an dem autoklavierbaren Sterilbehälter 30
- 36: photoelektrisches Bauelement an dem RFID-Etikett 34 an dem autoklavierbaren Sterilbehälter 30 zum Empfang optischer Leistung und zum Bereitstellen elektrischer Leistung für das RFID-Etikett 34
- 40: erster Bereich zum Identifizieren eines medizinischen Instruments 10
- 42: erste Kamera, zum optischen Erfassen eines medizinischen Instruments 10 auf der Ablagefläche 18
- 44: RFID-Leseeinrichtung für den ersten Bereich 40
- 50: zweiter Bereich zum Bestücken eines Instrumententrägers 10
- 52: zweite Kamera, zum Erfassen eines medizinischen Instruments 10 auf dem autoklavierbaren Instrumententräger 20
- 54: RFID-Schreibeinrichtung für den zweiten Bereich 50
- 56: erste Lichtquelle, zur Bereitstellung von Lichtleistung für das photoelektrische Bauelement 26 an dem RFID-Etikett 24
- 58: Projektionseinrichtung zum Projizieren des Umrisses 28 des medizinischen Instruments 10 in einer Soll-Position und Soll-Orientierung auf den autoklavierbaren Instrumententräger 20
- 60: dritter Bereich zum Befüllen eines Sterilbehälters 30
- 62: Kamera zum Erfassen eines medizinischen Instruments 10 auf dem autoklavierbaren Instrumententräger 20 in dem Sterilbehälter 30
- 64: RFID-Schreibe- und Leseeinrichtung für den dritten Bereich 60
- 66: zweite Lichtquelle, zur Bereitstellung von Lichtleistung für das photoelektrische Bauelement 36 an dem RFID-Etikett 34 an dem autoklavierbarer Instrumententräger 20
- 68: dritte Lichtquelle, zur Bereitstellung von Lichtleistung für das photoelektrische Bauelement 36 an dem RFID-Etikett 34 an dem autoklavierbarer Sterilbehälter 30
- 70: Computer als Einrichtung zur Bildauswertungseinrichtung
- 72: Eingabeeinrichtung als Teil einer Benutzerschnittstelle; insbesondere Tastatur
- 74: Ausgabeeinrichtung als Teil einer Benutzerschnittstelle; insbesondere Bildschirm
- 76: Datenbank
- 82: Leistungsversorgungseinrichtung des RFID-Etiketts 22, 32
- 84: Mikrocontroller des RFID-Etiketts 22, 32
- 86: Datenspeicher des RFID-Etiketts 22, 32
- 88: Sende- und Empfangseinrichtung des RFID-Etiketts 22, 32
- 101: Schritt (optisches Erfassen eines Bilds eines oder mehrerer medizinischer Instrumente)
- 102: Schritt (Erzeugen eines Bildsignals, das das erfasste Bild repräsentiert)
- 103: Schritt (Übertragen des Bildsignals zu einer Bildauswertungseinrichtung)
- 104: Schritt (Auswerten des Bildsignals durch die Bildauswertungseinrichtung hinsichtlich Identität, Anordnung des oder der medizinischen Instrumente und Erzeugen von Identitätsdaten und Anordnungsdaten)
- 105: Schritt (Übertragen der Identitätsdaten und der Anordnungsdaten)
- 106: Schritt (Speichern der Anordnung als Soll-Anordnung)
- 111: Schritt (optisches Erfassen eines Bilds eines oder mehrerer medizinischer Instrumente)
- 112: Schritt (Erzeugen eines Bildsignals, das das erfasste Bild repräsentiert)
- 113: Schritt (Übertragen des Bildsignals zu einer Bildauswertungseinrichtung)
- 114: Schritt (Auswerten des Bildsignals durch die Bildauswertungseinrichtung 70 hinsichtlich der Identität des medizinischen Instruments und Erzeugen von Identitätsdaten)
- 116: Schritt (Ermitteln der Soll-Anordnung des einen oder der mehreren identifizierten medizinischen Instrumente 10)
- 118: Schritt (Projizieren des oder der medizinischen Instrumente 10 in der ermittelten Soll-Anordnung auf den Instrumententräger 20)
- 121: Schritt (optisches Erfassen eines weiteren Bilds eines oder mehrerer medizinischer Instrumente 10 auf einem Instrumententräger 20)
- 122: Schritt (Erzeugen eines weiteren Bildsignals, das das erfasste weitere Bild repräsentiert)
- 123: Schritt (Übertragen des weiteren Bildsignals zu einer Bildauswertungseinrichtung)
- 124: Schritt (Auswerten des weiteren Bildsignals durch die Bildauswertungseinrichtung 70 hinsichtlich der Identität, Anordnung und Orientierung des oder der medizinischen Instrumente und Erzeugen von Identitätsdaten)
- 126: Schritt (Lesen der Soll-Bestückung des identifizierten Instrumententrägers 20 und der Soll-Anordnung des oder der medizinischer Instrumente 10 auf dem Instrumententräger 20)
- 127: Schritt (Vergleichen der ermittelten Bestückung mit der gelesenen Soll-Bestückung und der ermittelten Anordnungen mit der gelesenen Soll-Anordnung)
- 128: Schritt (Schritt (Erzeugen eines Meldesignals)
- 135: Schritt (Übertragen der Identitätsdaten zu einem mit dem Instrumententrägers 20 dauerhaft verbundenen Datenspeicher 86)
- 136: Schritt (Speichern der Identitätsdaten in dem mit dem Instrumententrägers 20 dauerhaft verbundenen Speicher 86)
- 140: Schritt (Einbringen des Instrumententrägers 20 in einen Sterilbehälter 30)
- 141: Schritt (optisches Erfassen eines weiteren Bilds eines oder mehrerer medizinischer Instrumente 10 auf dem Instrumententräger 20 in dem Sterilbehälter 30)
- 142: Schritt (Erzeugen eines weiteren Bildsignals, das das erfasste weitere Bild repräsentiert)
- 143: Schritt (Übertragen des weiteren Bildsignals zu einer Bildauswertungseinrichtung)
- 144: Schritt (Auswerten des weiteren Bildsignals durch die Bildauswertungseinrichtung 70 hinsichtlich der Identität, Anordnung und Orientierung des oder der medizinischen Instrumente und Erzeugen von Identitätsdaten)
- 146: Schritt (Lesen der Soll-Bestückung des identifizierten Instrumententrägers 20 und der Soll-Anordnung des oder der medizinischer Instrumente 10 auf dem Instrumententräger 20)
- 147: Schritt (Vergleichen der ermittelten Bestückung mit der gelesenen Soll-Bestückung und der ermittelten Anordnungen mit der gelesenen Soll-Anordnung)
- 148: Schritt (Schritt (Erzeugen eines Meldesignals)
- 154: Schritt (Lesen der Identitätsdaten aus der mit dem Instrumententräger 20 dauerhaft verbundenen Dateneinrichtung 24
- 155: Schritt (Übertragen der Identitätsdaten zu einem mit einem Sterilbehälter 30 dauerhaft verbundenen Datenspeicher 86)
- 156: Schritt (Speichern der Identitätsdaten in dem mit dem Sterilbehälter 30 dauerhaft verbundenen Speicher 86)
- 160: Schritt (Bereitstellen des Instrumententrägers 20 für eine medizinische Maßnahme)
- 161: Schritt (optisches Erfassen eines weiteren Bilds eines oder mehrerer medizinischer Instrumente 10 auf dem Instrumententräger 20)
- 162: Schritt (Erzeugen eines weiteren Bildsignals, das das erfasste weitere Bild repräsentiert)
- 163: Schritt (Übertragen des weiteren Bildsignals zu einer Bildauswertungseinrichtung)
- 164: Schritt (Auswerten des weiteren Bildsignals durch die Bildauswertungseinrichtung 70 hinsichtlich der Identität und o, Anordnung und Orientierung des oder der medizinischen Instrumente und Erzeugen von Identitätsdaten )
- 166: Schritt (Lesen der Soll-Bestückung aus einem mit dem Instrumententräger 20 dauerhaft verbundenen Speicher 86)
- 167: Schritt (Vergleichen der ermittelten Bestückung mit der gelesenen Soll-Bestückung)
- 168: Schritt (Setzen des Attributs "unsteril" zu einem medizinischen Instrument 10, wenn der Vergleich der Identitätsdaten ergibt, dass das medizinische Instrument 10 nicht mehr an dem Instrumententräger 20 angeordnet ist)
- 168: Schritt (Erhöhen eines Zählers, der die Verwendungen des medizinischen Instruments zählt, um eins)

## Patentansprüche

1. System zur Unterstützung der Handhabung medizinischer Instrumente (10), mit:
einer Kamera (42, 52, 62) zum optischen Erfassen eines Bilds eines oder
mehrerer medizinischer Instrumente (10) und zum Bereitstellen eines Bildsignals, das das erfasste Bild repräsentiert;
einer Bildauswertungseinrichtung (70) zum Empfangen des Bildsignals, zum Identifizieren von einem oder mehreren in dem durch das Bildsignal repräsentierten Bild abgebildeten medizinischen Instrumenten (10) und zum Bereitstellen von Identifikationsdaten, die die abgebildeten Instrumente (10) identifizieren;
einer Datenübertragungseinrichtung (56, 66) zum Senden von Identifikationsdaten an eine Datenempfangseinrichtung (88) einer mit einem autoklavierbaren Instrumententräger (20) dauerhaft verbundenen Dateneinrichtung (22).

2. System gemäß dem vorangehenden Anspruch, ferner mit:
einem autoklavierbaren Instrumententräger (20) zum Bevorraten,
Transportieren und Bereithalten medizinischer Instrumente (10);
einer mit dem autoklavierbaren Instrumententräger (20) dauerhaft verbundenen und autoklavierbaren Dateneinrichtung (22) mit einer
Datenempfangseinrichtung (88) zum Empfangen von Daten und einem wiederholt beschreibbaren und auslesbaren Speicher (86) zum Speichern von durch die Datenempfangseinrichtung (88) empfangener Daten.

3. System gemäß dem vorangehenden Anspruch, ferner mit:
einer mit dem autoklavierbaren Instrumententräger (20) dauerhaft verbundenen und autoklavierbaren Leistungsempfangseinrichtung (24) zum Empfangen von Leistung in Form von Licht und zum Bereitstellen elektrischer Leistung für die Dateneinrichtung (22).

4. System gemäß dem vorangehenden Anspruch, ferner mit:
einer Lichtquelle (56, 66) zum Bereitstellen von Leistung in Form von Licht für die mit dem autoklavierbaren Instrumententräger dauerhaft verbundene Leistungsempfangseinrichtung (24).

5. System gemäß einem der vorangehenden Ansprüche, ferner mit:
einer Projektionseinrichtung (58) zum sichtbaren Projizieren einer vorgesehenen Soll-Position eines medizinischen Instruments (10) auf eine Oberfläche des autoklavierbaren Instrumententrägers (20).

6. System gemäß einem der vorangehenden Ansprüche, ferner mit:
einer Datenbank (76), in der vorgesehene Positionen medizinischer Instrumente (10) auf einer Oberfläche des autoklavierbaren Instrumententrägers (20) gespeichert sind.

7. Autoklavierbarer Instrumententräger (20) zum Bevorraten, Transportieren und Bereithalten medizinischer Instrumente (10), mit:
einer mit dem autoklavierbaren Instrumententräger (20) dauerhaft verbundenen und autoklavierbaren Dateneinrichtung (24) mit einem wiederholt beschreibbaren und auslesbaren Speicher (86) und einer Datenübertragungseinrichtung (88) zum Empfangen und Senden von Daten;
einer mit dem autoklavierbaren Instrumententräger (20) dauerhaft verbundenen und autoklavierbaren Energieempfangseinrichtung (26) zum Empfangen von Leistung in Form von Licht und zum Bereitstellen elektrischer Leistung für die Dateneinrichtung (22).

8. Autoklavierbarer Instrumententräger (20) gemäß dem vorangehenden Anspruch, ferner mit:
einem Barcode oder einem anderen ein- oder zweidimensionalen optisch lesbaren Code (22) oder einer anderen optisch lesbaren Einrichtung zur Identifizierung des autoklavierbaren Instrumententrägers (20).

9. Autoklavierbarer Sterilbehälter (30) zum Bevorraten, Transportieren und Bereithalten eines oder mehrerer Instrumententräger (20) mit jeweils einem oder mehreren medizinischen Instrumenten (10), mit:
einer mit dem autoklavierbaren Sterilbehälter (30) dauerhaft verbundenen und
autoklavierbaren Dateneinrichtung (34) mit einem wiederholt beschreibbaren und auslesbaren Speicher (86) und einer Datenübertragungseinrichtung (88) zum Senden und Empfangen von Daten;
einer mit dem autoklavierbaren Sterilbehälter (30) dauerhaft verbundenen und autoklavierbaren Energieempfangseinrichtung (36) zum Empfangen von Leistung in Form von Licht und zum Bereitstellen elektrischer Leistung für die Dateneinrichtung (32).

10. Autoklavierbarer Sterilbehälter (30) gemäß dem vorangehenden Anspruch, ferner mit:
einem Barcode oder einem anderen ein- oder zweidimensionalen optisch lesbaren Code (32) oder einer anderen optisch lesbaren Einrichtung zur Identifizierung des autoklavierbaren Sterilbehälters (30).

11. Autoklavierbarer Instrumententräger (20) gemäß einem der Ansprüche 7 und 8 oder autoklavierbarer Sterilbehälter (30) gemäß einem der Ansprüche 9 und 10, bei dem
die Energieempfangseinrichtung (26, 36) eine photovoltaische Zelle oder einen optoelektrischen Wandler auf der Basis von Galliumarsenid umfasst.

12. Verfahren zur Unterstützung der Handhabung medizinischer Instrumente (10), mit folgenden Schritten:
optisches Erfassen (101; 111; 121; 141) eines Bilds eines oder mehrerer medizinischer Instrumente (10);
Erzeugen (102; 112; 122; 142) eines Bildsignals, das das erfasste Bild repräsentiert;
Übertragen (103; 113; 123; 143) des Bildsignals zu einer Bildauswertungseinrichtung (70);
Auswerten (104; 114; 124; 144) des Bildsignals durch die Bildauswertungseinrichtung (70), um das oder die in dem Bild abgebildeten medizinischen Instrumente (10) zu identifizieren und Erzeugen von Identitätsdaten, die die Identitäten des oder der identifizierten medizinischen Instrumente (10) repräsentieren;
Übertragen (135) von Identitätsdaten;
Speichern (136) der erzeugten Identitätsdaten in einem mit einem Instrumententräger (20) dauerhaft verbundenen Speicher (86) oder Vergleichen (125) der erzeugten Identitätsdaten mit Identitätsdaten, die in dem mit dem Instrumententräger (20) dauerhaft verbundenen Speicher (86) gespeichert sind.

13. Verfahren gemäß dem vorangehenden Anspruch, bei dem das Auswerten (104) des Bildsignals durch die Bildauswertungseinrichtung (70) ein Erfassen der Anordnung der in dem Bild abgebildeten medizinischen Instrumente (10) umfasst,
ferner mit folgendem Schritt:
Speichern (106) der erfassten Anordnung als Soll-Anordnung der medizinischen Instrumente (10).

14. Verfahren gemäß Anspruch 12, ferner mit folgenden Schritten:
Ermitteln (116) einer Soll-Anordnung eines identifizierten medizinischen Instruments (10) auf einem Instrumententräger (20);
Projizieren (117) des medizinischen Instruments (10) in der Soll-Anordnung auf den Instrumententräger (20).

15. Verfahren gemäß einem der Ansprüche 12 bis 14, ferner mit folgenden Schritten:
optisches Erfassen (121; 141) eines weiteren Bilds des oder der medizinischen Instrumente (10) auf dem Instrumententräger (20);
Erzeugen (122; 142) eines weiteren Bildsignals, das das erfasste weitere Bild repräsentiert;
Übertragen (123; 143) des weiteren Bildsignals zu der
Bildauswertungseinrichtung (70);
Auswerten (124; 144) des weiteren Bildsignals durch die Bildauswertungseinrichtung (70), um die Anordnung des oder der in dem weiteren Bild abgebildeten medizinischen Instrumente (10) zu bestimmen;
Erzeugen (128; 148) eines Meldesignals abhängig davon, ob die bestimmte Anordnung des oder der medizinischen Instrumente (10) ihrer Soll-Anordnung entspricht.

16. Verfahren gemäß dem vorangehenden Anspruch, bei dem das Erzeugen (128; 146) des Meldesignals ferner davon abhängig ist, ob das Speichern (136; 156) der Identitätsdaten des oder der identifizierten medizinischen Instrumente (10) in dem mit dem Instrumententräger (20) dauerhaft verbundenen Speicher (86) erfolgreich war.

17. Verfahren gemäß dem vorangehenden Anspruch, ferner mit folgendem Schritt: Setzen (167) des Attributs "unsteril" zu einem medizinischen Instrument (10), wenn der Vergleich der Identitätsdaten ergibt, dass das medizinische Instrument (10) nicht mehr an dem Instrumententräger (20) angeordnet ist.

18. Verfahren gemäß einem der Ansprüche 12 bis 16, ferner mit folgenden Schritten:
Einbringen (140) des Instrumententrägers (20) in einen Sterilbehälter (30);
Auslesen (154) der Identitätsdaten des oder der identifizierten medizinischen Instrumente (10) aus dem mit dem Instrumententräger (20) dauerhaft verbundenen Speicher (86);
Übertragen (155) der Identitätsdaten des oder der identifizierten medizinischen Instrumente (10) zu einem mit dem Sterilbehälter (30) dauerhaft verbundenen Speicher (86);
Speichern (156) der Identitätsdaten des oder der identifizierten medizinischen Instrumente (10) in dem mit dem Sterilbehälter (30) dauerhaft verbundenen Speicher (86).
